# EUROPEAN PATENT SPECIFICATION

(11) **EP 1 785 490 B1**
(45) Date of publication and mention of the grant of the patent: **14.11.2007**
(21) Application number: 05090287.3
(22) Date of filing: 17.10.2005
(51) Int. Cl.: C12Q 1/68

(54) **Method for determining an unknown PNA sequence and uses thereof**
Verfahren zur Bestimmung einer unbekannten PNS Seqenz und seine Verwendungen
Procédé pour la détermination d'une séquence PNA inconnue et ses utilisations

(43) Date of publication of application: 16.05.2007
(73) Proprietor: RiNA Netzwerk RNA-Technologien GmbH, 14195 Berlin (DE); PLS-Design GmbH, 20255 Hamburg (DE)
(72) Inventor: Bredehorst, Reinhard, Prof.Dr., 20255 Hamburg (DE); Glökler, Jörn, Dr., 10827 Berlin (DE); Grunwald, Thomas, Dr., 22459 Hamburg (DE); Matzas, Mark, Dipl.-Chem., 20259 Hamburg (DE); Spillner, Edzard, Dr., 22765 Hamburg (DE)
(74) Representative: Jungblut, Bernhard Jakob

(56) References cited:
- EP-A- 0 639 607
- US-A1- 2004 191 812
- US-B1- 6 399 364
- US-B1- 6 562 575

## Description

### Field of the invention.

The invention is related to a method for determining an unknown PNA sequence information and to methods for the selection, identification, accumulation and amplification of PNA molecules or producing PNA molecules capable of forming PNA/target molecule complexes.

### Background of the invention and state of the art.

Single stranded nucleic acids like RNA or ssDNA are capable of forming a threedimensional structure comparable with folding of proteins, which is essentially determined by the nucleic acid sequence. These threedimensional structure are based mainly on intramolecular base pair interactions and promote the capability to recognize the surface structure of target molecules and to bind to target molecules having surface structures matching with the threedimensional structure of the nucleic acid. Such nucleic acids capable of binding to target molecules due to threedimensional matching are called aptamers. The target molecules may be of any species like small organic molecules (e.g. coffein), large organic molecules (e.g. synthetic polymers), peptides, proteins, enzymes, amino acids, saccharides, nucleotides, oligo- and polynucleotides, hormones, polysaccharides, or surface structures of cells or viruses. Aptamers may even be capable to distinguish between enantiomers and to recognise the presence or absence of functional groups like methyl- or hydroxyl groups. Accordingly, aptamers may be used in particular in therapeutic and/or diagnostic applications (Jayasena, S.D., Aptamers: An emerging class of molecules that rival antibodies in diagnostics., Clin. Chem. 1999, 45(9), 1628-50; Herman, T. and Patel, D.J., Adaptive recognition by nucleic acid aptamers., Science 2000, 287(5454), 820-5).

In order to identify aptamers capable of binding to a defined target molecule it is known to apply selection methods based on providing a nucleic acid library, which is then contacted with target molecules of a defined target molecule species. Binding nucleic acids are amplified and accumulated until the concentration of binding nucleic acids is sufficient for cloning and/or sequencing. With the sequence information obtained the aptamers for the defined target molecule species may be generated in production scale and used for the intended application. One such method is the so-called SELEX method described in the publications Tuerk, C., Gold, L., Systematic evolution of ligands by exponential enrichment: RNA ligands to bacteriophage T4 DNA polymerase., Science 1990, 249(4968), 505-10 and Ellington, A.D. and Szostak, J.W., In vitro selection of RNA molecules that bind specific ligands. Nature 1990, 346(6287), 818-22.

Peptide nucleic acids (PNA) are synthetic molecules which are capable of hybridizing with oligomeric or polymeric nucleic acids. PNA molecules resemble such nucleic acids but differ in the backbone chain being constituted of a peptidic achiral structure based on N-(2-aminoethyl)glycine monomers instead of ribose-phosphate monomers. The natural bases are thereby attached to the backbone chain by carboxymethylene units. Remarkably, the backbone chain of PNA molecules is electrically neutral, whereas the backbone chain of nucleic acids is charged. Further, PNA molecules are resistent against degradation by nucleases, proteases or peptidases. Finally, PNA molecules have shown a comparatively low toxicity in biological systems, if any. Additional reference is made to the publication McMahon, B.M. et al., Pharmacokinetics and tissue distribution of a peptide nucleic acid after intravenous administration, Antisense Nucleic Acid Drug Dev. 2002, 12(2), 65-70.

Accordingly, it would be favorable to obtain and identify PNA molecule species, which function like aptamers, in particular for therapeutic and/or diagnostic applications or which provide other certain properties including but not limited to catalytic functions, since PNA molecules are highly stable in biological environments and lack a charge which makes a number of target molecules accessible for PNA aptamers that are eluded from selection processes involving natural nucleic acids due to unspecific binding based on electrostatic interactions. One possibility to obtain such a PNA molecule species with binding properties or other functional properties would be to bring a library with PNA molecules of a plurality of different PNA molecule species in contact with a desired target molecule and separate the unbound PNA from the PNA/target molecule complexes. One major problem herein is the identification of obtained PNA molecules with the desired properties, since the amount of PNA will not be sufficient for analysis by weight measurement or structure determination using standard methods (e.g. mass spectrometry or spectroscopy). Therefore the selected PNA molecule species need to be amplified prior to analysis. However, presently there are no technologies existing providing means for the amplification of PNA or their sequencing. Because of this reason methods like SELEX are not applicable for PNA hence they involve an amplification of the library components.

Attempts to transfer of PNA sequence information to natural nucleic acids have been reported in Schmidt, J.G., Information transfer from peptide nucleic acids to RNA by template-directed synthesis., Nucleic Acids Res. 1997, 25(23), 4797-802. However the low efficiency of these chemical coupling procedures make these methods unapplicable to practical processes. As a result these technology has been pursued during the last years, because there is an urgent need in the field for a method that allows amplification of PNA with reasonable efficiency within an acceptable period of time. Furthermore there is need in the field for a process to select, amplify and sequence PNA molecules which function like aptamers or provide other functional properties.

It is known in the art how to sequence nucleic acids (Sanger F. et al., DNA-sequencing with chain-terminating inhibitors., Proc. Nat. Acad. Sci. 1977, 74, 5463-5467 and Hunkapiller, T. et al., Large-scale and automated DNA sequence determination., Science 1991, 254, 59-67). However these processes are not transferable to PNA due to the different backbone chain chemistry.

Synthesis of PNA per se is further known by using amplified DNA as template (Rosenbaum, D.M. and Liu, D.R., Efficient and sequence-specific DNA-templated polymerization of peptide nucleic acid aldehydes. J. Am. Chem. Soc. 2003, 125(46), 13924-5) or by sequencing the obtained nucleic acid bearing the PNA sequence information and subsequent chemical synthesis of PNA by standard methods (Christensen, L. et al., Solid-phase synthesis of peptide nucleic acids., J. Pept. Sci. 1995, 1(3), 175-83 and Thomson, S.A. et al., Fmoc mediated synthesis of peptide nucleic acids., Tetrahedron 1995, 51, 6179-94).

US-6,399,364 discloses a method in which the steps of hybridizing probes with a target, separating bound probes from unbound probes by coupling target to solid phase, releasing bound probes from targets and sequencing the probes in order to determine the sequence of the target are carried out. US 2004/191812 A1 discloses the use of a library of PNA molecules for selecting aptamers in a SELEX process.

Technical problem of the invention.

It is a first object of the invention to provide means for sequencing PNA oligomers or polymers.

It is a further object of the invention is to provide means to select and determine the sequence of PNA oligomers or polymers capable of binding a defined target molecule.

It is a further object of the invention to provide means for identifying, accumulating and/or producing PNA molecules capable of binding to a defined target molecule.

It is a further object of the invention to provide means for effectively separating unbound PNA molecules from PNA/target molecule complexes.

It is a further object of the invention to provide PNA molecules, which bind with high affinity to defined target molecules.

Summary of the invention and preferred embodiments.

For achieving the first mentioned object, the invention provides a method for determining the sequence of PNA molecules of a specific PNA molecule species, wherein PNA molecules are contacted with one or several different nucleic acid molecule species comprising nucleic acid molecules with at least one nucleotide, wherein the nucleic acid molecules at least partially comprise a nucleic acid sequence that is complementary to at least a partial sequence of the PNA molecule, wherein nucleic acid molecules having complementary sequences bind to the PNA molecules forming nucleic acid/PNA hybrids, wherein nucleic acid molecules with non-complementary sequences are separated from the nucleic acid/PNA hybrids, wherein nucleic acid molecules with non-complementary sequences are optionally degraded enzymatically, wherein thereafter the nucleic acid/PNA hybrids are dissociated into single stranded nucleic acid molecules and PNA molecules, wherein the single stranded nucleic acid molecules are subjected to a sequencing process providing sequence information about the single stranded nucleic acid sequence, and wherein the sequence information is optionally translated into the complementary PNA sequence information.

The invention is based on the fact that PNA may hybridize with nucleic acids with high affinity and stringency and that hybridizing nucleic acids may be sequenced using standard processes, thereby obtaining sequence information being complementary to the sequence of the PNA.

The sequence length of the PNA may be at least 5, preferably at least 10, more preferably at least 15.

The sequence length of the nucleic acid molecules may be at least 2, preferably at least 3, more preferably at least 4. The method of the invention can be carried out in a variety of embodiments which differ basically in the sequence length of the nucleic acid molecules contacted with the unknown PNA molecule.

In one embodiment the sequence length of the nucleic acid molecules is at least the sequence length of the PNA molecules, wherein binding of complementary PNA molecules and nucleic acid molecules is carried out under hybridization conditions forming the nucleic acid/PNA hybrids without ligation of nucleic acid molecules bound to the PNA molecules. In this most simple embodiment the nucleic acid molecule may be longer than the PNA molecule, wherein the overhang sequences may e.g. be used for amplification of the nucleic acid molecules after dissociation, for immobilization or for detection using standard routines.

The invention may further comprise means for specific enzymatical degradation of non-hybridized oligonucleotides. Examples for the enzymatical degradation are the use of nucleases (e.g. DNaseI), use of single-strand specific nucleases (e.g. but not restricted to S1 nuclease and Mungobean nuclease), use of restriction enzymes either of type II (e.g. XbaI, HindIII etc.) or of type IIs (e.g. MlyI, BseRI etc.). Within the following examples further details of some of the degradation processes may be taken.

In practice it will be favorable to first obtain or create a nucleic acid library, wherein the nucleic acid molecules or partial sequences thereof are randomized. The randomized part should have a length corresponding to the sequence length of the PNA under investigation (this is either known, since the PNA may origin from a PNA library, or may be determined using standard methods for molecular weight measurement).

In another embodiment the sequence length of the PNA molecules is greater than a total sequence length or a randomized partial sequence length of the nucleic acid molecules, preferably by a factor of 2 or more, more preferably by a factor of 3 or more, most preferably by a factor of 4 or more, wherein nucleic acid molecules binding adjacent to each other to the PNA molecules are ligated chemically or enzymatically, preferably enzymatically, forming the nucleic acid/PNA hybrid. Within this embodiment the total sequence length of the nucleic acid molecules may be larger that the sequence length of the PNA provided that the randomized sequence length is as above. If the randomized sequence length is one half or more than one half of the sequence length of the PNA molecules, then typically two different nucleic acid molecules of the randomized library will bind adjacent to each other. Then ligation must be performed between these two nucleic acid molecules. The overhangs may, again, be used for amplification, immobilization, detection, etc.

In another embodiment, the total sequence length of the nucleic acid molecules comprised in the randomized nucleic acid library may be less than the sequence length of the PNA, wherein the total sequence is randomized. Then two, three, four, five, six, seven, eight, nine, ten or more nucleic acid molecules will bind adjacent to each other to the PNA. Then n-1 ligation reactions are carried out (n = number of binding nucleic acid molecules) for making the hybrid.

In another embodiment, the total length of the nucleic acid molecules is one, i.e. the nucleic acid molecule library comprises a mixture of all nucleic acid monomers. Again n-1 ligation reaction must be carried out for making the hybrid.

The ligation reactions may be carried out with any process known in the art, although enzymatical ligation is preferred. Examples for chemical ligation reactions are the cyanogen bromide (CNBr) process and the 1-(3-[dimethylamino]propyl)-3-ethylcarbodiimide hydrochloride (EDC) process.

With respect to the enzymatic ligation, it is preferred to carry this out using a ligation enzyme selected from the group consisting of "DNA ligase I, DNA ligase II, DNA ligase III, DNA ligase IV, DNA ligase V, T4 DNA ligase, Taq DNA ligase, T4 RNA ligase, T4 RNA ligase II, ThermoPhage^{™} single-stranded DNA ligase, Rma DNA ligase, Tsc DNA ligase, *E.coli* DNA ligase, LdMNPV DNA ligase, LigTK, Mth ligase, PBCV-1 DNA ligase, Pfu DNA ligase, Sealase, T4 ATP ligase, Vaccinia ligase, Tfi DNA ligase, Tth DNA ligase, Band IV, DREL, gp24.1, P52, RM378 RNA ligase, TbMP52, Rcllp, DNA ligase D, XRCC4-ligase, T7 DNA ligase, Bst ligase, DraRnl", preferably using T4 RNA ligase. Within the following examples further details of some of the standard processes may be taken. Standard processes for enzymatic ligation reactions are well known to the skilled artisan and need not be described here in detail.

The basic process of selection and identification according to the invention includes the following steps:
a) in a solution comprising at least one target molecule species and PNA molecules of a plurality of different PNA molecule species, PNA molecules of a PNA species with a binding affinity to the target molecule form PNA/target molecule complexes with the target molecules of the target species;
b) separation of the plurality of non-bound PNA molecule species from the PNA/target molecule complexes is effected;
c) isolation of the PNA molecule species with binding affinity to the target molecule from the PNA/target molecule complex and determination of the PNA sequence is effected.
d) determination of the PNA sequence

If the amount of selected PNA molecules with binding affinity to the target molecule is too low for sequencing the selected PNA molecules are amplified prior to sequencing.

If the selection efficiency of the process is too low to sufficiently reduce the presence of PNA molecules which are not binding to the target molecule an additional round of selection can be performed as described in the following:
a) in a solution comprising at least one target molecule species and PNA molecules of a plurality of different PNA molecule species, PNA molecules of a PNA species with a binding affinity to the target molecule form PNA/target molecule complexes with the target molecules of the target species;
b) separation of the plurality of not bound PNA molecule species from the PNA/target molecule complexes is effected;
c) Isolation of the PNA molecule species with binding affinity to the target molecule from the PNA/target molecule complex;
d) amplification of the sequence information of the PNA molecules with binding affinity to the target molecule is effected;
e) contacting amplified PNA molecules with binding affinity to the target molecules with target molecules as described in (a), separating the PNA molecules as described in (b), and isolating the PNA molecule species with binding affinity to the target molecule from the PNA/target molecule complex as described in (c);
f) either continuing the process as described in (d) for further rounds of selection or proceeding to step (g);
g) Determination of the PNA sequence

The invention further provides means to identify PNA molecules with certain properties like binding affinities or catalytic properties either after a single selection step and subsequent identification of selected PNA molecules wherein the sequence information from PNA is transferred to nucleic acids, RNA or DNA. This provides the possibility of amplifying the sequence information of the PNA by amplification of the obtained nucleic acid with standard methods (Mullis, K.B. and Faloona, F.A., Specific synthesis of DNA in vitro via a polymerase-catalysed chain reaction., Methods Enzymol. 1987, 155, 335-50) and their subsequent sequencing, whereas the obtained sequence can be translated to complementary PNA sequence information. This information can be used for a chemical de novo synthesis of PNA molecules capable of binding a defined target molecule or providing other properties.

Alternatively the amplified DNA can be used for synthesis of PNA molecules in a template directed manner which equals an amplification of PNA molecules for a subsequent selection round.

The invention may further comprise means for the separation of nucleic acid/PNA hybrids from non-hybridized nucleic acids in the order of magnitude of at least 10E4, preferably 10E6, more preferably 10E8, most preferably 10E10 by affinity based methods using affinity tags connected either to the PNA or to the nucleic acid, selected from the group consisting of e.g. "biotin-streptavidin system, strep-tag, digoxigenin, His-tag etc." and a matrix selected from the group consisting of e.g. "agarose, sepharose, magnetic beads etc.", carrying the corresponding binding partner for the affinity tag, wherein these methods also involve the use of specifically cleavable functional groups in the linker chains to the affinity tags selected from the group consisting of e.g. "reductive cleavable disulfide groups, photolabile groups, pH-sensitive groups etc." for the specific elution of nucleic acid/PNA hybrids from the matrix.

The invention may further comprise means to separate nucleic acid/PNA hybrids from non-hybridized nucleic acids in the order of magnitude of at least 10E4, preferably 10E6, more preferably 10E8, most preferably 10E10 using chromatographic or electrophoretic methods selected from the group consisting of "HPLC, ion-chromatography, capillary electrophoresis, free flow electrophoresis, capillary gel electrophoresis, micellar electrokinetic capillary chromatography, capillary electrochromatography, non-gel-sieving, affinity-capillary-electrophoresis, capillary ion electrophoresis, HPLC, LC, ion-chromatography".

As well the separation methods as the degradation methods can be combined with any methods for generating PNA complementary DNA mentioned above.

It will be favorable if the single stranded hybrid nucleic acid molecules are amplified, preferably using PCR, prior to the sequencing process.

The invention further comprises means for the amplification of generated complementary DNA by a variety of methods concerning different methods for generation of primer hybridization sites either by ligation of terminal linker oligonucleotides at the 5'- and/or the 3'-terminus of generated complementary nucleic acids, wherein methods are involved to reduce background arising by directly ligated linker oligonucleotides using restriction endonucleases, or by use of nucleic acid/PNA chimera wherein nucleic acids in the chimera are elongated by terminal nucleotidyltransferases to provide primer hybridization sites, or by use of nucleic acid/PNA chimera, wherein nucleic acids in the chimera form hairpin loops to provide start-oligonucleotides being elongated by nucleic acid fragments and ligation, wherein hairpin loops can be cut by restriction endonucleases or by single-strand specific nucleases taken out of the group consisting of "S1 nuclease, Mungobean nuclease etc.". Within the following examples further details of some of the mentioned processes may be taken.

The invention further comprises a method for separating unbound PNA molecules from a solution comprising at least one target molecule species and PNA molecules of a plurality of different PNA molecule species, wherein PNA molecules of a PNA molecule species with a binding affinity to the target molecule species form PNA/target molecule complexes with the target molecules of this target molecule species, wherein the solution comprises an ionic compound effective to promote at least a partial electrical charge to either unbound PNA molecules or PNA/target molecule complexes, wherein the solution is subjected to an electrophoretic separation method comprising application of an electric field to the solution, and wherein PNA/target molecule complexes or unbound PNA molecules with a partial electrical charge promoted by the ionic compound obtain an electrical migration component of translation in the electrical field, thereby being separated from components in the solution having a different charge/size ratio or no electrical charge.

This aspect is based on the finding that ionic compounds in the solution comprising unbound PNA molecules and PNA/target molecule complexes are capable of associating with one of both groups only thereby imparting an electrical charge to the associated group. Typically, the ionic compound will promote at least a partial electrical charge to the PNA/target molecule complexes. This allows separation methods based on application of an electrical field, to which PNA molecules are normally not sensitive due to the neutral backbone chain, in contrast to nucleic acids.

The ionic compound may be in particular an ionic detergent, preferably an ionic detergent selected from the group consisting of "benzethonium-chloride, benzethonium-hydroxide, cetylpyridinium-bromide, cetylpyridinium-chloride, cetyltrimethylammonium-bromide,cetyltrimethylammonium-chloride, (2-hydroxyethyl)trimethylammonium salts, denatoniumbenzoates, denatoniumsaccharides, dodecyl-sulfates (preferably sodium-dodecyl-sulfate (SDS) but also lithium-dodecyl-sulfate and ammonium-dodecyl-sulfate), hexadecyltrimethylammoniumbromide (CTAB), hexadecyltrimethylammonium-chloride (CTAC), lauroylsarcosine, N,N-dimethyldecylamine-N-oxide (DDAO), N,N-dimethyldodecylamine-N-oxide (LDAO), sodium-bis(2-ethylhexyl)-sulfosuccinate, butanesulfonates, chenodeoxycholates, cholates, decanesulfonates, deoxycholates (DOC), sodium-docusate, dodecanesulfonates, glycocholates, glycodeoxycholates, heptanesulfonates, hexadecanesulfonates, octanesulfonates, octylsulfates, propanesulfonates, taurochenodeoxycholates, taurocholates, taurodehydrocholates, taurodeoxycholates, taurolithocholates, tauroursodeoxycholates, tetradecylsulfates, tert-octyl-phenyl-propanesulfonic-acid (TOPPS), Triton X-100, 3-(Cyclohexylamino)-1-propanesulfonic acid (CAPS), 4'-amino-7-benzamido-taurocholic acid (BATC)" and preferably sodium dodecylsulfate (SDS), or a mono-, bi-, or tri-nucleotide taken from the group consisting of e.g. but not restricted to "adenosine, cytosine, guanine, thymine, uracile, desoxyadenosine, desoxycytosine, desoxyguanine, desoxythymine", or oligonucleotides respectively desoxyoligonucleotides with a length of five, preferably four, more preferably three, most preferably two monomers.

The separation method may be selected from the group consisting of "capillary electrophoresis, capillary gel electrophoresis, micellar electrokinetic capillary chromatography, capillary electrochromatography, non gel sieving, affinity capillary electrophoresis, capillary ion electrophoresis, HPLC, LC, ion chromatography", preferably is capillary electrophoresis. For a review of electrophoretic separation methods and details of such methods, which are common in the art reference is made to e.g Righetti, P.G., Electrophoresis: the march of pennies the march of dimers, J. Chromatogr. A 2005, 1079(1-2), 24-40.

The concentration of the ionic compound in particular detergents may be below the Critical Micellary Concentration (CMC), preferably is below 8 mmol/l, more preferably is below 5 mmol/l, even preferably is in the range between 0,01 - 3 mmol/l, most preferably is in range between 0,1 - 1 mmol/l.

Definitions.

A target molecule species may be any species and is not restricted to the group consisting of "small organic molecules, peptides, proteins, enzymes, amino acids, hormones, polysaccharides, and surface structures of cells or viruses". In particular, the target molecule species may as well be non-biological and comprise any molecular structure for which a need of detection exists, e.g. explosives, environmentally dangerous substances, and the like.

A PNA molecule consists of a backbone chain of at least two monomers, wherein to each of the monomers carries one nucleotid bases adenine, cytosine, guanine, uracile or thymine. A PNA molecule is capable of hybridizing with a oligo- or polynucleic acid of natural structure. The monomers in one PNA molecule may be different or the same. Preferably the monomers are the same and N-(2-Aminoethyl)glycine, wherein the nucleotide bases are attached to the backbone chain via carboxymethylene units.

Unbound PNA molecules are molecules that are not bound to a target molecule. Preferably unbound PNA molecules are also not bound to any other compound present in the solution.

A molecule species is defined by a singular specific chemical structure. All molecules of a molecule species have the identical specific chemical structure. Preferably all molecules of a molecule species further have the same three-dimensional structure in space.

The molecules of different molecule species differ from each other in the three-dimensional structure and, optionally, in the chemical structure.

A PNA molecule binds to a target molecule with a binding affinity, if the three-dimensional structure of the PNA molecule fits to the three-dimensional structure of the target molecule of to a partial structure thereof. Typical affinity values are better than 10 µM.

A PNA/target molecule complex typically does not involve covalent bonds, but rather hydrogen bonds, electrostatic, hydrophobic and/or Van-der-Waals interactions.

The sequence length of a PNA molecule is defined as the number of backbone monomers present in the molecule.

Accumulation of a molecule species comprises increasing the concentration of the molecule species in a solution and/or increasing the number of the molecules of the species in a sample.

A PNA library comprises PNA molecules of a plurality of different PNA molecule species. The PNA molecule species typically differ in the sequence of the nucleotide bases attached to the backbone chain only.

Different nucleic acid species differ in the sequence of the nucleotide bases attached to the backbone chain.

Hybridization between PNA molecules and nucleic acid molecules takes place by hydrogen bonding between pairs of nucleotide bases present on the nucleic acid and the PNA if the sequences of the nucleotide bases of the PNA and of the nucleic acid match. Preferably the match is a complete and a 100% match to the shorter of both, if any.

The amplification of nucleic acids comprises increasing the number of identical nucleic acids in a sample, preferably by PCR methods known to the skilled artisan.

In the following the invention and components thereof the are described in non-limiting examples.

### Example 1: Separation of PNA and PNA/target molecule complexes by capillary electrophoresis

The separation of PNA and a complex of dihydrofolate reductase (DHFR) with PNA bound thereto is performed using a covalently polyacrylamide-coated capillary with an internal diameter of 75 µm, an overall length of 70 cm and an effective length (injection site to detector) of 51 cm. The separation buffer containes 20 mmol/l disodium hydrogenphosphate adjusted to pH 7.3 and at least 0.2 mmol/l sodiumdodecylsulfate (SDS). The applied voltage is 430 V/cm (anode at detector side). The capillary is temperated at 28°C by airflow. Samples are injected hydrodynamically for 5 s and detected by absorption at 200 nm.

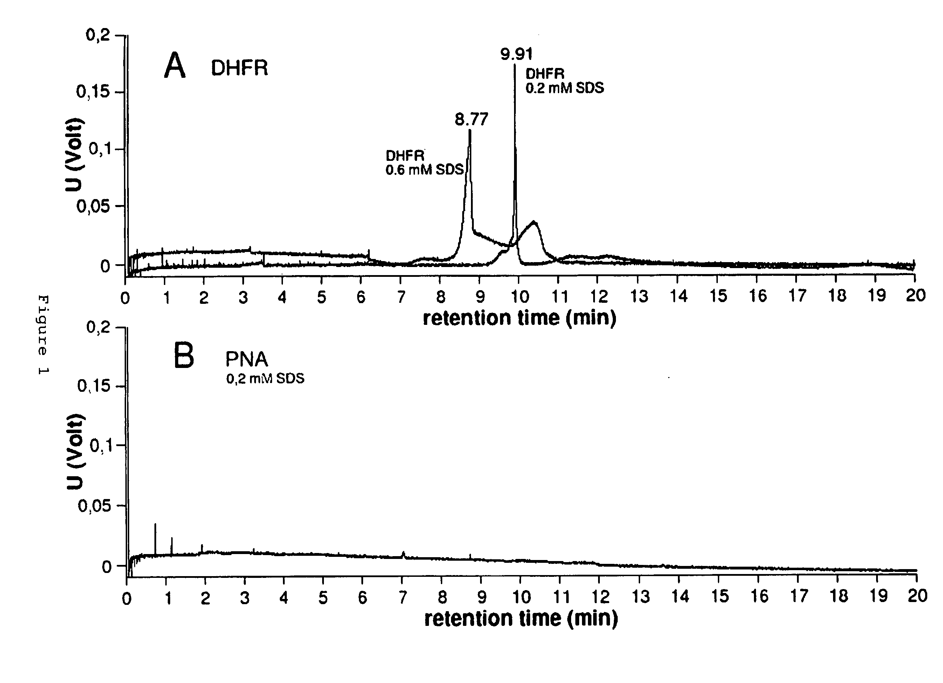

Figure 1A shows the separation of a mixture of 10 µmol/l randomized 18mer PNA and 4 mg/ml DHFR in separation buffer containing either 0.6 mmol/l or 0.2 mmol/l SDS. In this experiment the presence of DHFR/PNA complexes has not been tested for, but the experiments are, nevertheless representative for the separation of unbound PNA from complexes since uncomplexed DHFR and complexed DHFR will behave essentially identically.

Using 0.6 mmol/l SDS DHFR could be detected after 8.8 min, using 0.2 mmol/l DHFR could be detected after 9.9 min. Higher concentrations of SDS (but below critical micellar concentration) had no significant effect on the migration speed. PNA could not be detected at all using this separation conditions. Figure 1B shows the verification of slow respectively no migration of PNA. This was performed by injection of PNA exclusively whereas no signal could be detected in a time period of 20 min using the separation conditions mentioned above. The injected amount of PNA was sufficient to generate a voltage signal of 0.2 Volt which was verified by pushing the PNA through the capillary hydrodynamically (not shown).

### Example 2: Separation of PNA and PNA/target molecule complexes by HPLC

Separation of PNA and DHFR/PNA complexes by HPLC is performed by using C18 reverse phase matrix in a column with an internal diameter of 1 mm and a length of 10 cm (particle diameter = 2 µm). The mobile phases are 0.1 mol/l triethylammoniumacetate (pH 7) and acetonitrile at a flow rate of 40 µl/min. Substances are detected by absorption at 254 nm and 280 nm. The injected volume is 5 µl. Separation is started isocratically with 10 % acetonitrile. Under these separation conditions protein fractions are detected in a time period of 2 to 6 min after sample injection. However the free PNA remains on the column and can be eluted and discarded together with all sample components by increasing the acetonitrile concentration in the mobile phase within a single step to 90 %.

### Example 3: Synthesis of a PNA oligomer or polymer and creation of a PNA library.

The synthesis of a 18-mer PNA with the sequence CCG ATT AAC GCT TGT ACC C is carried out using the synthesis processes described in the reference Thomson, S.A. et al., Fmoc mediated synthesis of peptide nucleic acids., Tetrahedron 1995, 51, 6179-94. In the same manner, different PNA 18-mers, differing in the nucleotide base sequence only, are synthesized in the same manner. The different PNA 18-mers are then joined in a solution forming the PNA library.

### Example 4: Incubation of the PNA library with a target molecule and separation of the complexes from unbound PNA.

A solution comprising the PNA library according to the example 3 is contacted with DHFR under conditions preferably similar to those present in later applications for the selected PNA molecules and which are predominantly similar to that used in common SELEX experiments that are described in the references Tuerk, C., Gold, L., Systematic evolution of ligands by exponential enrichment: RNA ligands to bacteriophage T4 DNA Polymerase., Science 1990, 249(4968), 505-10 and Ellington, A.D. and Szostak, J.W., In vitro selection of RNA molecules that bind specific ligands. Nature 1990, 346(6287), 818-22. Then the solution is subjected to capillary electrophoresis or HPLC as a separation process according to example 1 or 2. Thereafter the isolated complexes are dissociated into DHFR and PNA either by incubation at high temperature, by pH-shift, competitive (by addition of existing ligands) or alternatively by degradation of DHFR using proteases or proteinases (e.g. Proteinase K).

### Example 5: Hybridization of PNA with an oligonucleotide library and complementary nucleic acid amplification

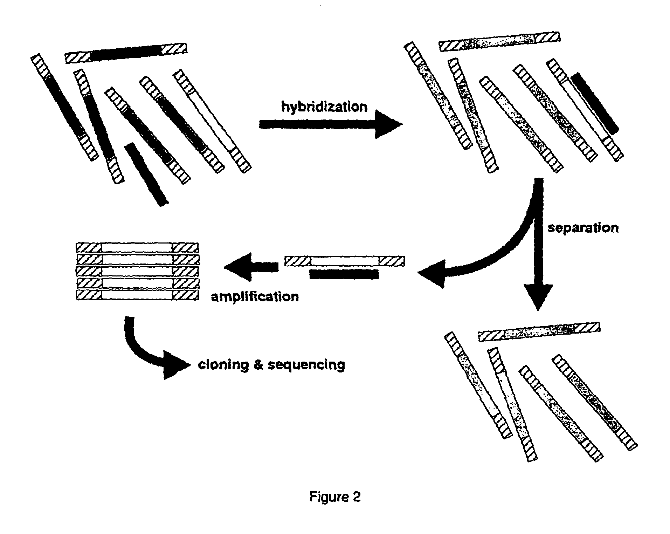

Isolation of complementary ssDNA from an oligonucleotide library was performed by hybridization of matching oligonucleotides to a biotinylated 18mer PNA. 12 nmol of the oligonucleotide library (5'GAA TTC CAG ATC TCT NNN NNN NNN NNN NNN NNN GAT ATC AGG ATC CCA3') was dissolved in 120 µl of buffer (10 mmol/l disodium hydrogenphosphate, pH 7.5). Different amounts of PNA (at least 10⁵ molecules) were added to the reaction solution. The mixture was heated to 95°C and the tubes were placed into a thermal isolated container (dewar) filled with hot water (92°C). The container was isolated additionally with styrofoam and was cooled to room temperature over a time period of approximately five days. Generated PNA/DNA-hybrids were subsequently isolated by different methods and the DNA was amplified. Figure 2 shows the principle of PNA identification by hybridization to complementary ssDNA from a library with 18 randomized nucleotides (grey) and known primer regions (hatched). After hybridization of PNA (black) to matching sequences (blank) the remaining ssDNA has to be removed. Amplification of PNA-complementary PNA is performed by PCR using the known primer regions (hatched).

Isolation by PNA/DNA-Hybrids by immobilization on a solid phase and subsequent washing: 20 µl of an agarose matrix suspension with immobilized streptavidin was added to the hybridization solution containing the oligonucleotide library and PNA/DNA-hybrids. The mixture was shaked for 6 to 15 h and the suspension was transfered to a micro column (or alternatively into a filter pipet tip). The non hybridized DNA was removed by a flow of at least 100 column volumes of washing buffer (0.3 mol/l NaCl, 60 mmol/l Tris-HCl (pH 8.0), 2 mmol/l EDTA, 1 % (w/v) SDS) through the column. Afterwards the matrix was washed with 1 ml 10 mmol/l Tris-HCl (pH 8.5). The PNA/DNA-hybrids were cleaved from the matrix specifically by reducing the disulfide bond in the biotinylation linker on the PNA with 10 mmol/l Tris-HCl (pH 8.5), 20 mmol/l DTT for 2 h at room temperature. The solution can be prepared for amplification of DNA by gel filtration (Sephadex G-25).

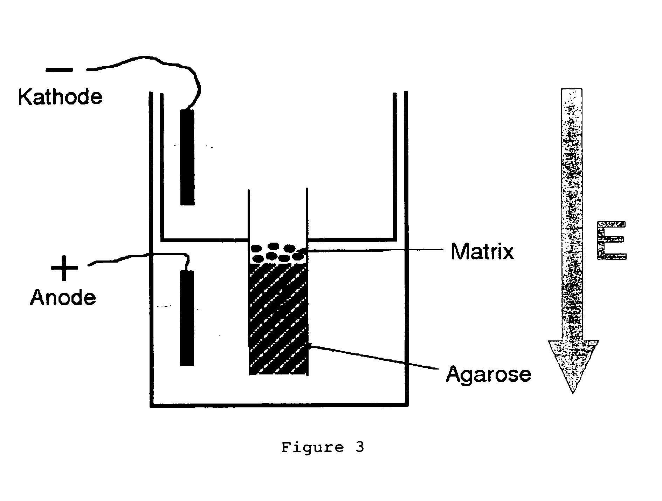

Isolation by PNA/DNA-Hybrids by immobilization on a solid phase and subsequent removal of free ssDNA in an electric field: 20 µl of an agarose matrix suspension with immobilized streptavidin (streptavidin coated magnetic beads can be used alternatively) was added to the hybridization solution containing the oligonucleotide library and PNA/DNA-hybrids. The mixture was shaked for 6 to 15 h and the suspension was transfered to a buffer (TBE) filled tube with 1 % solid agarose in the bottom part. The agarose matrix with bound PNA/DNA-hybrids is transferred into the tube to settle on the agarose. The tube with an internal diameter of 5 mm was fixed between two buffer reservoirs and an electric field with a voltage difference of 100 V was applied over the tube for 1 to 5 hours leading to a migration of the free DNA into the agarose and subsequently into the anode buffer reservoir. Afterwards the matrix was washed with 1 ml 10 mmol/l Tris-HCl (pH 8.5). The PNA/DNA-hybrids were cleaved from the matrix specifically by reducing the disulfide bond in the biotinylation linker on the PNA with 10 mmol/l Tris-HCl (pH 8.5), 20 mmol/l DTT for 2 h at room temperature. The solution can be prepared for amplification of the DNA by gel filtration (Sephadex G-25). This separation method can also be applied in addition to preceding washing procedures. Figure 3 shows a schematic representation of the apparatus for the removal of non hybridized ssDNA using an electric field. PNA/DNA-Hybrids immobilized on agarose matrix were separated from free ssDNA with an applied voltage that leads to migration of ssDNA into the solid agarose in the column which avoids the diffusion of ssDNA back into the upper buffer reservoir.

Specific enzymatic degradation of non hybridized ssDNA with S1 nuclease: The specific degradation of non hybridized ssDNA with S1 nuclease was performed in 10 mmol/l Tris-acetic acid (pH 8.3), 50 mmol/l potassium acetate, 5 mmol/l magnesium acetate, 1 µg/ml BSA, 0.01 % (v/v) Tween 20. Samples contained PNA (N-CCG ATT AAC GCT TGC ACC-C) and the oligonucleotides Pos2(3)D2-1rev (5'ATT TAT GAG GAG TCC GGT GCA AGC GTT AAT CGG GAT ATC AGG ATC CCA3'), Pos3(3)rev (5'GGA CTC CTC ATA AAT3'), Pos4 (5'TGG GAT CCT GAT ATC3') in concentrations of 1 µmol/l each. The reactions were incubated for 2 min at 90°C and afterwards slowly cooled to room temperature. After addition of S1 nuclease (50 or 100 Units) samples were incubated 3 h at 20°C.

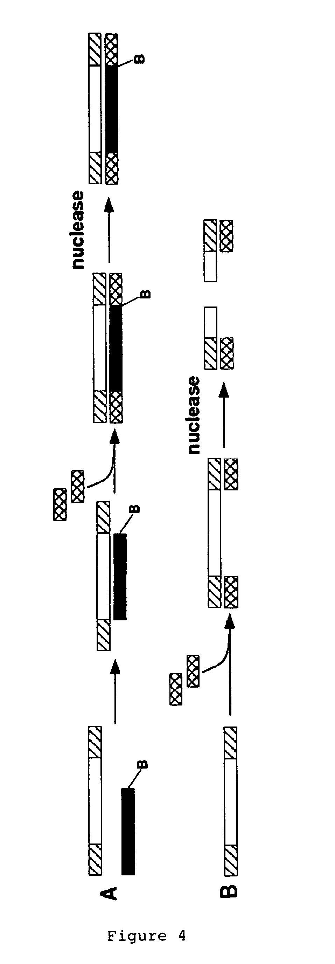

Figure 4 shows the principle of specific degradation of non hybridized ssDNA by single-strand specific nucleases. PNA (filled) protects the central part (blank) and complementary oligonucleotides (cross-hatched) protect the primer regions (hatched) of ssDNA against hydrolysis by single-strand specific nucleases (A). If no matching PNA is present, the middle region can be degraded (B).

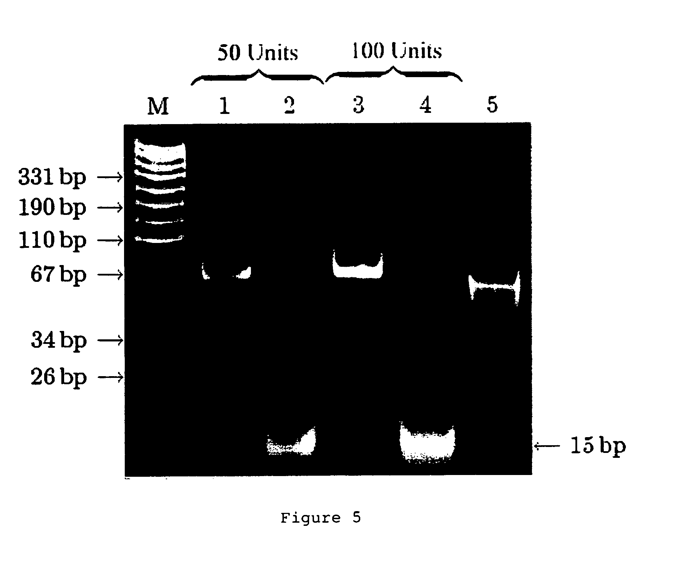

Figure 5 shows an experimental evaluation of the specific degradation of ssDNA with S1 nuclease. Samples with ssDNA and equimolar amounts of PNA and protection oligonucleotides for the primer regions the DNA is protected against degradation with 50 respectively 100 units S1 nuclease (lanes 1 and 3). Without PNA the single-stranded segments are hydrolyzed (lanes 2 and 4). Only the double-stranded primer regions remain intact. (lane 1: with PNA, 50 units S1 nuclease; lane 2: without PNA, 50 U S1 nuclease; lane 3: with PNA, 100 Units S1 nuclease; lane 4: without PNA, 100 U S1 nuclease; lane 5: without PNA, no nuclease; M: DNA-standard, pUC19/MspI) Separation of DNA was performed in 15 % polyacrylamide (29:1, acrylamide:bisacrylamide) under native conditions.

Specific enzymatic degradation of non hybridized ssDNA with Mungobean nuclease: The specific degradation of non hybridized ssDNA with Mungobean nuclease was performed in 10 mmol/l Tris-acetic acid (pH 8.3), 50 mmol/l potassium acetate, 5 mmol/l magnesium acetate, 1 µg/ml BSA, 0.01 % (v/v) Tween 20 (Zn²⁺ present in enzyme storage buffer). Samples contained PNA (N-CCG ATT AAC GCT TGC ACC-C) and the oligonucleotides Pos2(5)D2-1rev (5'ATT CTA TCA CGA GTC GGT GCA AGC GTT AAT CGG GAT ATG AGG ATC CCA3'), Pos3(5)rev (5'GAC TCG TGA TAG AAT3'), Pos4(5) (5'TGG GAT CCT CAT ATC3') in concentrations of 1 µmol/l each. The reactions were incubated for 2 min at 90°C and slowly cooled to room temperature. After addition of Mungo bean nuclease (20 or 10 Units) samples were incubated 15 h at 20°C.

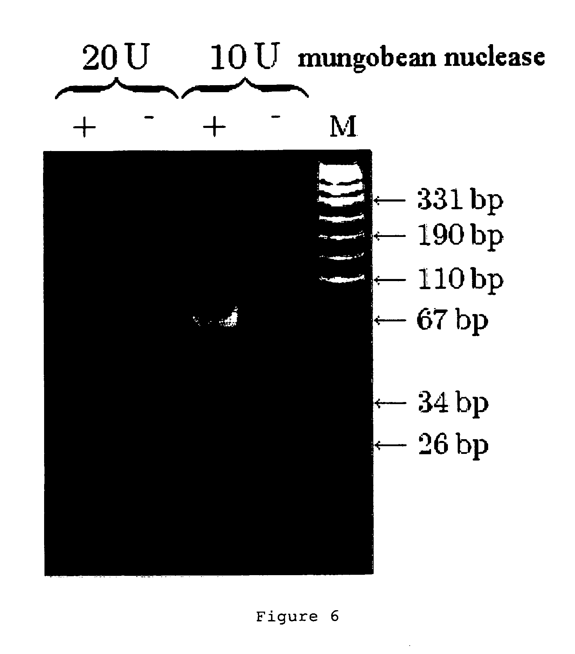

Figure 6 shows an experimental evaluation of the specific degradation of ssDNA with Mungobean nuclease. Samples with ssDNA and equimolar amounts of PNA are protected against degradation with 20 respectively 10 units Mungobean nuclease (lanes assigned with +). Without PNA the single-stranded segments are hydrolyzed (lanes assigned with -). (+: with PNA; -: without PNA; M: DNA-standard, pUC19/MspI) Separation of DNA was performed in 15% polyacrylamide (29:1, acrylamide:bisacrylamide) under native conditions.

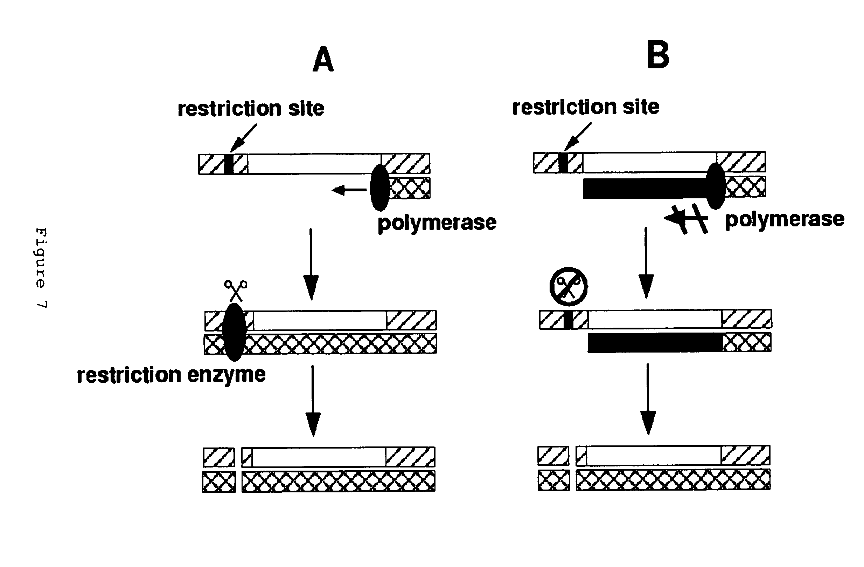

Specific enzymatic degradation of non hybridized ssDNA with Type II restriction enzymes: The ssDNA contains a restriction site for a restriction enzyme Type II in the primer regions (Figure 7; hatched) of the ssDNA library. Hence the restriction enzyme is only able to hydrolyze dsDNA, restriction can only be performed after the backward primer (cross-hatched) has been elongated by a DNA polymerase (black ellipsoide) (A). Reaction is executed in a primer elongation mix prior to temperature cycling for amplifiction. When the primer elongation is blocked by a hybridized PNA (filled black) in the middle region (blank) the DNA remains single stranded at the restriction site and no hydrolyzation takes place (B).

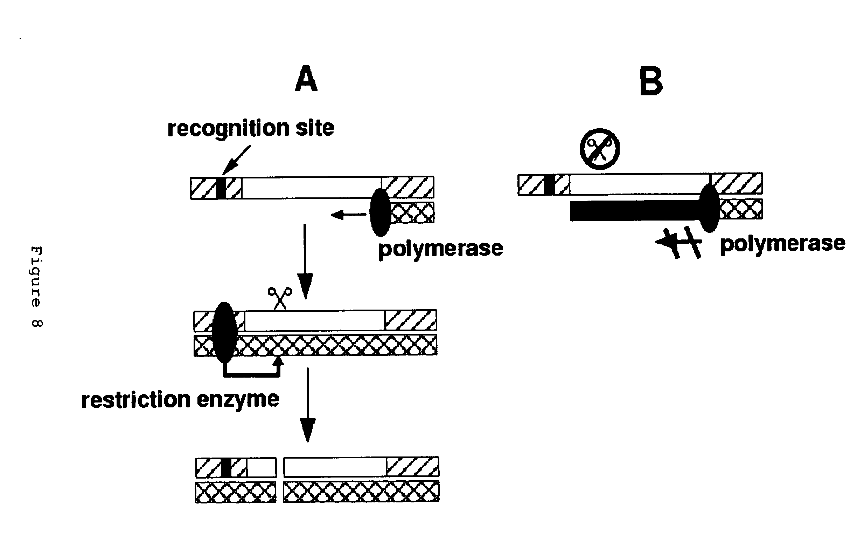

Specific enzymatic degradation of non hybridized ssDNA with Type IIs restriction enzymes: The sequence of ssDNA contains a recognition site for a Type IIs restriction enzyme in the primer regions of the ssDNA library (Figure 8; hatched). The restriction of the DNA takes place in the hybridization site for the PNA (blank) and can be blocked by a hybridized PNA (filled black). The reaction is executed in a primer elongation mix prior to temperature cycling. Polymerases (black ellipsoide) elongate the primer (cross-hatched) producing dsDNA which can be cut by restriction enzymes in the recognition site (A). PNA (B; filled black) blocks the primer elongation by polymerases, the DNA remains single-stranded and will not be hydrolysed by the enzyme (B). Function of the specific restriction was performed in 10 mmol/l Tris-acetic acid (pH 8.3), 30 mmol/l potassium acetate, 5 mmol/l magnesium acetate, 1 µg/ml BSA, 0.01 % (v/v) Tween 20. The reactions contained additionally 200 µmol/l dNTPs (each) and 8 U/ml DeepVentR(exo⁻)-DNA-Polymerase (NEB), PNA (N-CCG ATT AAC GCT TGC ACC-C) and the oligonucleotides Pos2(3)D2-lrev (5'ATT TAT GAG GAG TCC GGT GCA AGC GTT AAT CGG GAT ATC AGG ATC CCA3') and Pos4 (5'TGG GAT CCT GAT ATC3') in concentrations of 1 µmol/l. All components of the reaction except the restriction enzyme were heated 2 min to 90°C and cooled to 41°C prior to addition of 5 or 10 Units MlyI and incubation for 15 h.

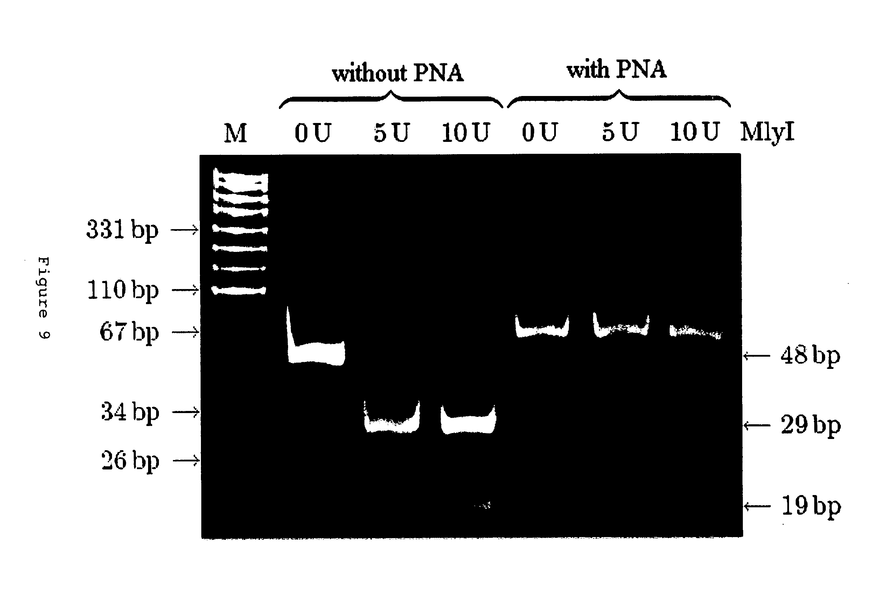

Figure 9 shows the PAGE analysis of the restriction products with PNA and without PNA with 0, 5 or 10 Units of the restriction enzyme MlyI. Restriction of the 48 bp DNA leads to fragments of 29 bp and 19 bp DNA. Presence of PNA blocks the restriction, DNA remains intact and has a higher apparent length due to the hybridized and uncharged PNA.

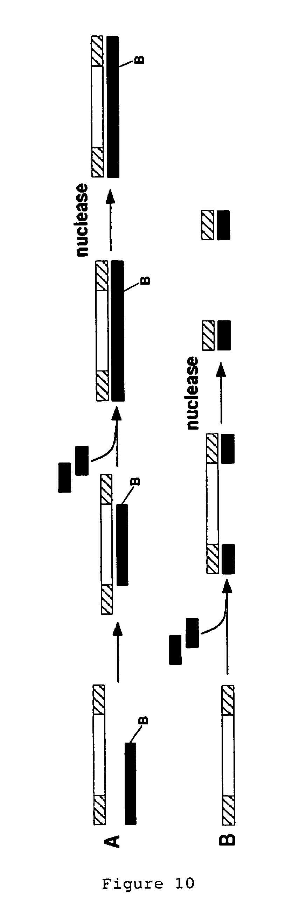

Specific enzymatic degradation of non hybridized ssDNA with non-sequence specific nucleases (e.g. DNaseI): Since a PNA/DNA-Hybrid is not a substrate for the most nucleases, the degradation of non hybridized ssDNA can be performed by incubation with DNases after protection of necessary primer regions with PNA. If there is a matching PNA (Figure 10, filled black) for the central region (A, blank) the primer regions (A, hatched) will be protected by corresponding PNA (A, black). Incubation with nuclease will destroy all regions of DNA not hybridized to a matching PNA (B).

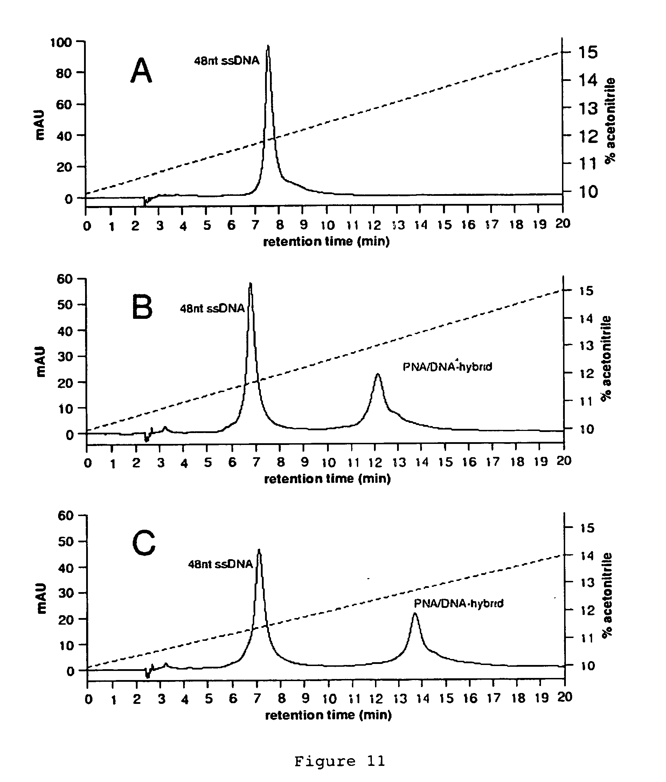

Separation of ssDNA and PNA/DNA-Hybrids with HPLC: HPLC separation of ssDNA and PNA/DNA-Hybrids was performed by using C4 reverse phase matrix in a column with an internal diameter of 4.6 mm and a length of 25 cm (particle diameter=5 µm). The mobile phases were 0.1 mol/l triethylammoniumacetate (pH 7) and acetonitrile at a flow rate of 1.3 ml/min. Substances were detected by absorption at 254 nm. Injection volume was 20 µl. Separation were executed with a gradient from 10 to 15 % acetonitrile over 20 min. Figure 11 shows chromatogramms for the separation of 48 nt ssDNA and PNA/DNA-hybrids using HPLC. Chromatogramm A shows the separation of a sample containing 48 nt ssDNA (5'GAA TTC CAG ATC TCT GGT GCA AGC GTT AAT CGG GAT ATC AGG ATC CCA3') exclusively. Chromatogramms C and D show separation of samples containing a mixture of 48 nt ssDNA (5'GAA TTC CAG ATC TCT GGT GCA AGC GTT AAT CGG GAT ATC AGG ATC CCA3') and 18mer PNA (N-CCG ATT AAC GCT TGC ACC-C) in a molar ratio of DNA:PNA / 2:1 using different solution gradients with 10-15% acetonitrile (A and B) and 10-14% acetonitrile (C). After separation, the DNA from PNA/DNA-hybrids could be amplified by PCR.

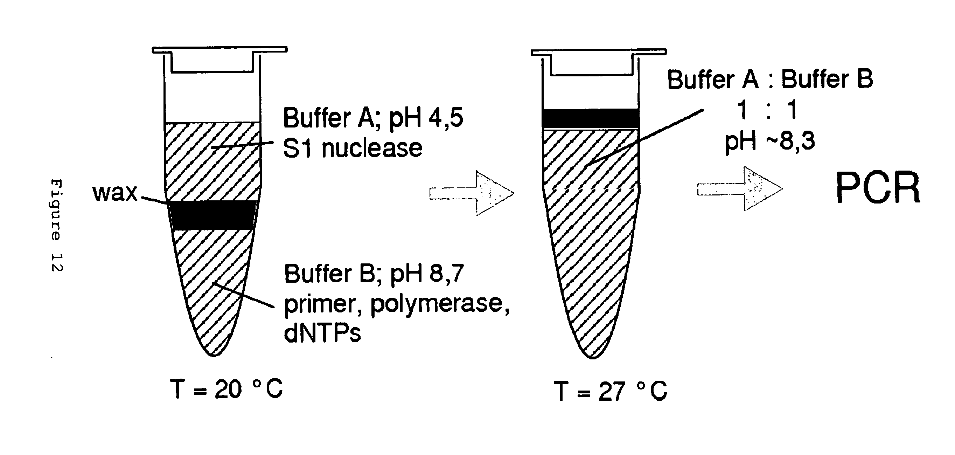

Buffer system with wax separation for degradation and subsequent amplification of ssDNA in a closed system: Two buffer compartments with a volume of 25 µl each were generated in a 200 µl reaction tube by separation with a wax layer. This provides the possibility of mixing the buffers automatically by rising the temperature and without intervention into the system by rising the temperature (Figure 12). The bottom compartment contained 40 mmol/l Tris-acetic acid (pH 8.7), 10 mmol/l potassium acetate, 5 mmol/l magnesium acetate, 10 mmol/l ammonium sulfate, 0.01 % (v/v) Tween 20, 2 µg/ml BSA, 4 mmol/l EDTA, 400 µmol/l dNTPs (each), 2 µmol/l of the primers Pos3(5) (5' ATT CTA TCA CGA GTC3') and Pos4(5) (5'TGG GAT CCT CAT ATC3') and 0.4 U DeepVentR(exo⁻)-DNA-Polymerase (NEB). After addition of 40 µl 25:1 (v/w) heptadecane/paraffin wax (Tₘ=73-80°C; Aldrich 411671-1) the tube is tempered at 20°C to solidify the wax. The solution for the top compartment containing 10 mmol/l potassium acetate (pH 4.6), 100 mmol/l NaCl, 100 µmol/l zinc sulfate, 5 mmol/l magnesium sulfate, 10⁵ hybrids of PNA (N-CCG ATT AAC GCT TGC ACC-C) and the oligonucleotide Pos2(5)D2-1rev (5'ATT CTA TCA CGA GTC GGT GCA AGC GTT AAT CGG GAT ATG AGG ATC CCA3'), 0.8 µmol/l of the oligonucleotides Pos3(5)rev (5'GGA CTC CTC ATA AAT3') and Pos4(5) (5'TGG GAT CCT CAT ATC3') can be given on the wax layer without mixing the buffer systems. Specific degradation of ssDNA in the top compartment can be initiated by addition of nuclease. After degradation the wax layer was melted by rising the temperature to 27°C for 5 min and mixing the compartments without intervention into the system. Essential metal ions for function of nucleases are bound to EDTA (which is present in the bottom compartment) as long as the dissociation constants of their corresponding EDTA compexes are lower than that of the magnesium-EDTA complex. Subsequent PCR (96°C primary denaturation; 35 cycles of 94°C, 45 s; 40°C, 1 min; 72°C, 30 s; final elongation 72°C, 1 min) was used to amplify DNA.

Immobilized nucleases for degradation of ssDNA and subsequent removal of enzymes: Using immobilized nucleases to degrade ssDNA specifically will ease the subsequent removal of the nucleases prior to PCR amplification.

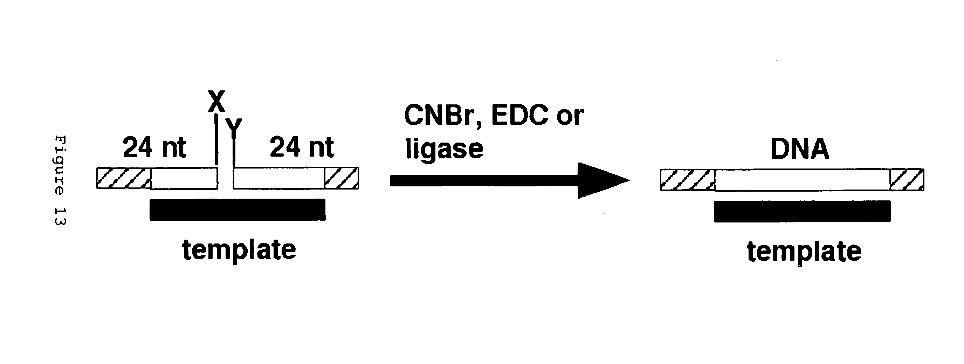

Chemical synthesis of PNA complementary DNA by using cyanogen bromide: For the chemical ligation of complementary DNA on a PNA template two 24 nucleotide long ssDNA were used that hybridized with nine nucleotides (Figure 13; blank) each on an 18mer PNA (filled black). To test different phosphorylation states either the oligonucleotide pair with a phosphorylation at the X-position (ApPos2(5)D2-lrev (5'ATT CTA TCA CGA GTC GGT GCA AGC-Pho3') and BPos2(5)D2-lrev (5'GTT AAT CGG GAT ATG AGG ATC CCA3')) or with a phosphorylation at the Y-position (APos2(5)D2-1rev (5'ATT CTA TCA CGA GTC GGT GCA AGC3') pBPos2(5)D2-1rev (5'Pho-GTT AAT CGG GAT ATG AGG ATC CCA3')) was used in the reaction. The chemical ligation with cyanogen bromide was performed in 250 mmol/l MES/triethylamin-buffer (pH 7.5) containing if so 20 mmol/l magnesium chloride. The samples contained further 10 pmol 18mer PNA (N-CCG ATT AAC GCT TGC ACC-C) or respectively 10 pmol of the oligonucleotide D2-1rev (5'GGT GCA AGC GTT AAT CGG3') as template and 10 pmol of each oligonucleotide of one pair in a volume of 9 µl. All reactions were heated to 94°C for 2 min and cooled slowly to 4°C. Ligation was initiated by addition of 1 µl 10 mol/l cyanogen bromide in acetonitrile. After 4 min incubation at 4°C samples were frozen immediately in liquid nitrogen and lyophilized. Residues where dissolved in 10 µl water for further analysis in a denaturating 15 % PAGE (29:1, acrylamide:bisacrylamide).

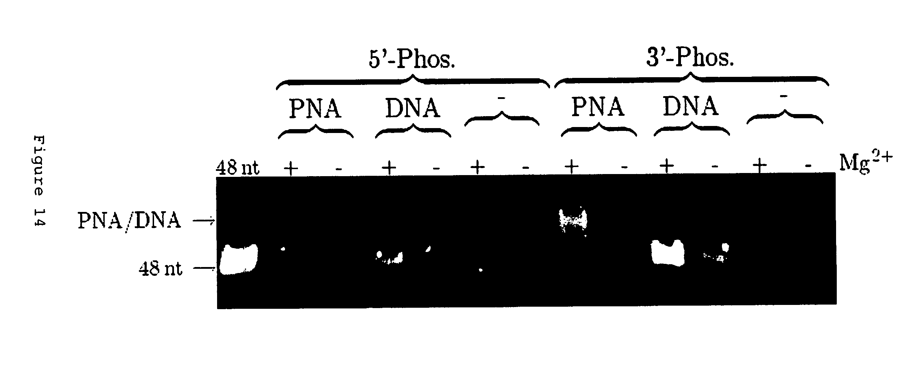

Figure 14 shows the PAGE analysis of the ligation reactions with phosphorylation either at the 5'- or at the 3'-end of adjacent DNA. It is obvious that higher yields were obtained in samples with magnesium-ions using ssDNA with 3'-phosphorylation and 5'-OH at juxtaposed ends and DNA as a template. However also with a PNA template detectable amounts of ligation products were obtained. (lanes assigned with "+": samples with 10 mmol/l magnesium chloride; PNA: samples containing a PNA template; DNA: samples containing a DNA template; -: samples without template)

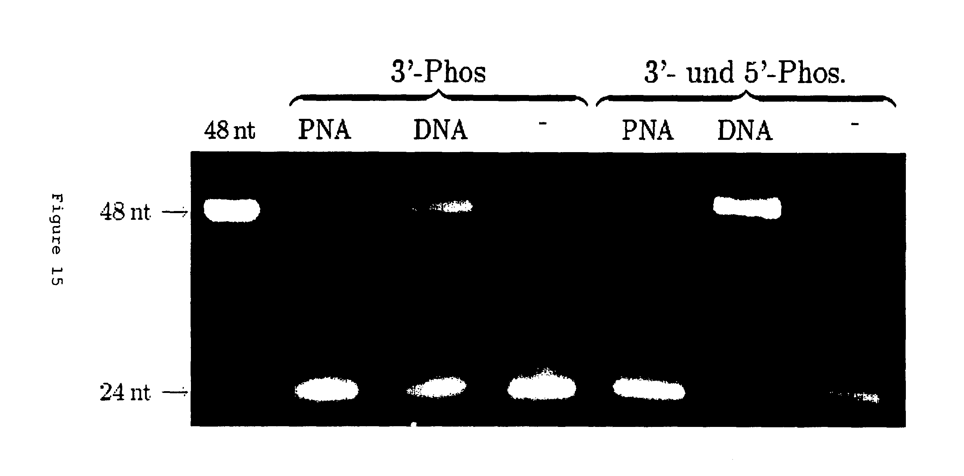

Chemical synthesis of PNA complementary DNA by using EDC: For the chemical synthesis of DNA on a PNA template two 24 nucleotide long ssDNA were used that hybridized with nine nucleotides each on an 18mer PNA (Figure 14). To test different phosphorylation states either the oligonucleotide pair ApPos2(5)D2-1rev (5'ATT CTA TCA CGA GTC GGT GCA AGC-Pho3') and BPos2(5)D2-1rev (5'GTT AAT CGG GAT ATG AGG ATC CCA3') or the oligonucleotide pair ApPos2(5)D2-1rev (5'ATT CTA TCA CGA GTC GGT GCA AGC-Pho3') pBPos2(5)D2-1rev (5'Pho-GTT AAT CGG GAT ATG AGG ATC CCA3') was used in the reactions. The chemical ligation using EDC was performed in 100 mmol/l MES/NaOH (pH 6) with 20 mmol/l magnesium chloride. The samples contained further 100 pmol 18mer PNA (N-CCG ATT AAC GCT TGC ACC-C) or respectively 100 pmol of the oligonucleotide D2-1rev (5'GGT GCA AGC GTT AAT CGG3') as template and 100 pmol of each oligonucleotide of a used oligonucleotide pair in a volume of 50 µl. All reactions were heated to 94°C for 2 min and cooled slowly to 10°C. Ligation was initiated by addition of 50 µl 400 mmol/l EDC solution and incubated for 19 h at 10°C. The subsequent denaturating PAGE analysis (15 % polyacrylamide; 29:1, acrylamide:bisacrylamide) is shown in Figure 15. (PNA: samples containing a PNA template; DNA: samples containing a DNA template; -: samples without template)

Template-directed coupling of PNA-hybridized ssDNA-fragments with ligases: Synthesis of PNA-complementary DNA by coupling ssDNA-fragments in a template-directed manner on a PNA can be realized by closing the nicks between hybridized fragments using ligases. These ligases could be single- or doublestranded (for example but not restricted to: DNA ligase I, DNA ligase II, DNA ligase III, DNA ligase IV, DNA ligase V, T4 DNA ligase, Taq DNA ligase, T4 RNA ligase, T4 RNA ligase II, ThermoPhage^{™} single-stranded DNA ligase, Rma DNA ligase, Tsc DNA ligase, E.coli DNA ligase, LdMNPV DNA ligase, LigTK, Mth ligase, PBCV-1 DNA ligase, Pfu DNA ligase, Sealase, T4 ATP ligase, Vaccinia ligase, Tfi DNA ligase, Tth DNA ligase, Band IV, DREL, gp24.1, P52, RM378 RNA ligase, TbMP52, Rcl1p, DNA ligase D, XRCC4-ligase, T7 DNA ligase, Bst ligase, DraRnl)

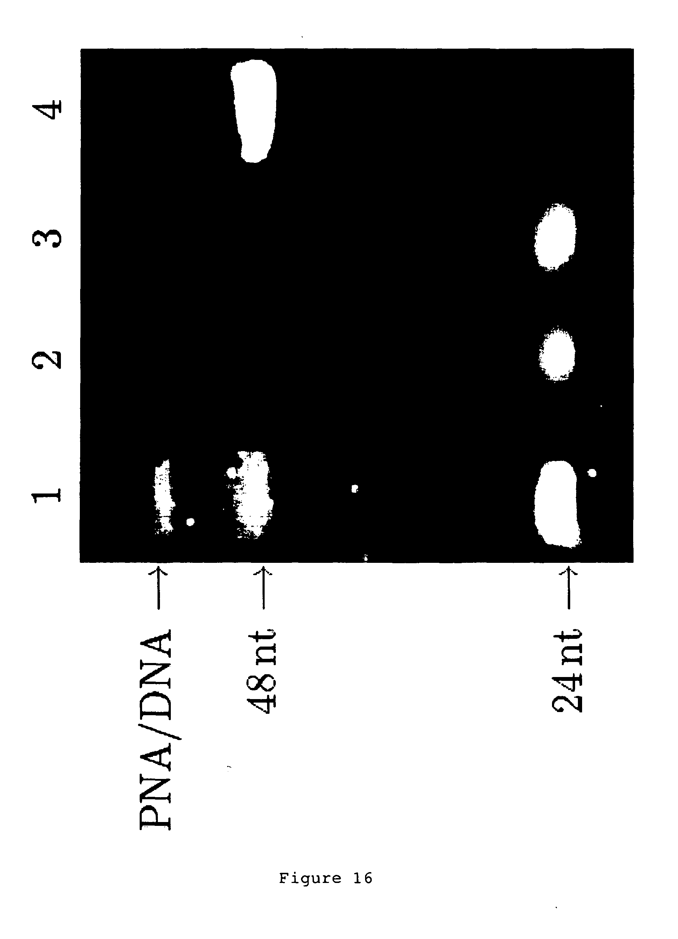

Enzymatical ligation of PNA complementary DNA by using T4 RNA Ligase and ssDNA with an overhang: The oligonucleotides APos2(5)D2-1rev (5'ATT CTA TCA CGA GTC GGT GCA AGC3') and pBPos2(5)D2-1rev (5'Pho-GTT AAT CGG GAT ATG AGG ATC CCA3') hybridized with nine nucleotides each on a template molecule that consisted of PNA (N-CCG ATT AAC GCT TGC ACC-C) and were ligated enzymatically with 500 U/ml T4 RNA ligase in 50 mmol/l HEPES/NaOH (pH 8), 10 mmol/l magnesium chloride, 100 µmol/l ATP and 10 µg/ml BSA. Concentrations for the oligonucleotides and the PNA were 1 µmol/l. All components except T4 RNA ligase and BSA were heated to 94°C for 2 min and cooled slowly to room temperature. Ligation reactions were started by addition of BSA and T4 RNA ligase prior to an incubation of 15 h at room temperature. Ligation products were analysed by a denaturating PAGE (15 % polyacrylamide; 29:1, acrylamide:bisacrylamide) which is shown in Figure 16. It is obvious that a ligation product of 48 nt has been obtained by using a PNA template (lane 1). No ligation product could be detected after using a DNA template (lane 2) or without any template (lane 3). Lane 4 contains a 48 nt ssDNA as reference.

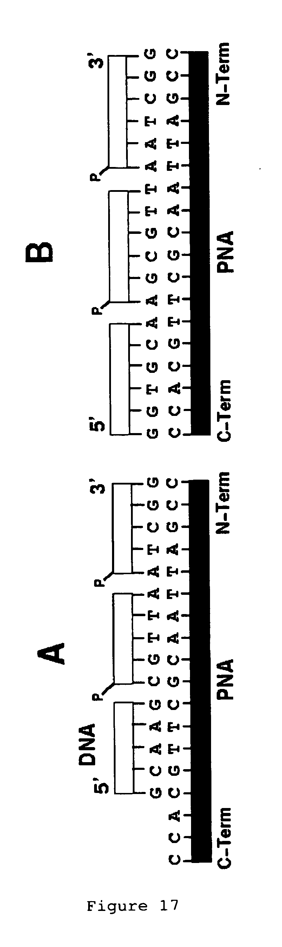

Enzymatical template-directed ligation of hexamer- and pentamer-ssDNA with T4 RNA ligase: Template-directed ligation of hexamer and pentamer ssDNA was realized in 50 mmol/l HEPES/NaOH (pH 8), 10 mmol/l magnesiumchloride, 100 µmol/l ATP and 10 µg/ml BSA with 330 U/ml T4 RNA ligase. The used oligonucleotides for hexamer ssDNA ligation were 6merA (5'Pho-GGT GCA3'), 6merB (5'Pho-AGC GTT3'), 6merC (5'Pho-AAT CGG3') (Figure 17B) and for pentamer ligation the oligonucleotides 5merA (5'GCA AG3'), 5merB (5'Pho-CGT TA3') and 5merC (5'Pho-ATC GG3') were used (Figure 17A). In a reaction either the hexamer or the pentamer oligonucleotides were present in concentrations of 2 µmol/l each. Additionally the reaction mixtures contained 2 µmol/l PNA (N-CCG ATT AAC GCT TGC ACC-C). All components except T4 RNA ligase and BSA were heated to 94°C for 2 min and cooled to room temperature prior to addition of T4 RNA ligase and BSA to start the reaction. After incubation for 15 h at room temperature the reaction mixtures were gel filtrated (Sephadex G-25) and the eluent volumes were reduced by lyophilization if necessary. Ligation products could be analysed on a denaturating 20 % PAGE (29:1, acrylamide:bisacrylamide) with 8 mol/l urea and 10 % formamide at ~50°C and subsequent silver staining.

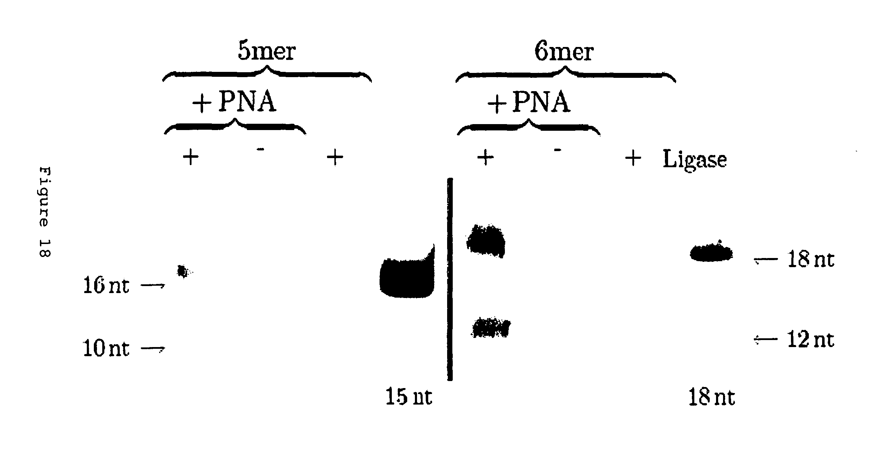

Ligation of pentamers (Figure 18; left part of gel) yielded 15 nt and 10 nt ssDNA, ligation of hexamers (right part of gel) yielded 18 nt and 12 nt ssDNA which resulted from ligation of three respectively two ssDNA fragments on a template. No product has been detected in samples without a template or without ligase (lanes assigned with "-"). 15 nt and 18 nt ssDNA served as reference in the gel. (+: samples containing ligase; -: samples without ligase; +PNA: samples containing a PNA template; 5mer: ligation with pentamer ssDNA; 6mer: ligation with hexamer ssDNA)

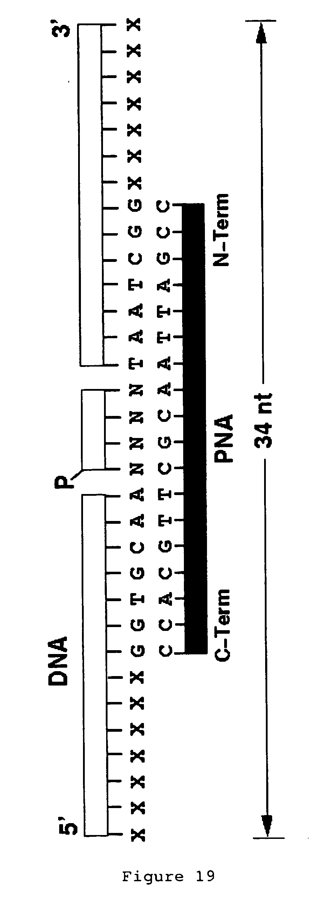

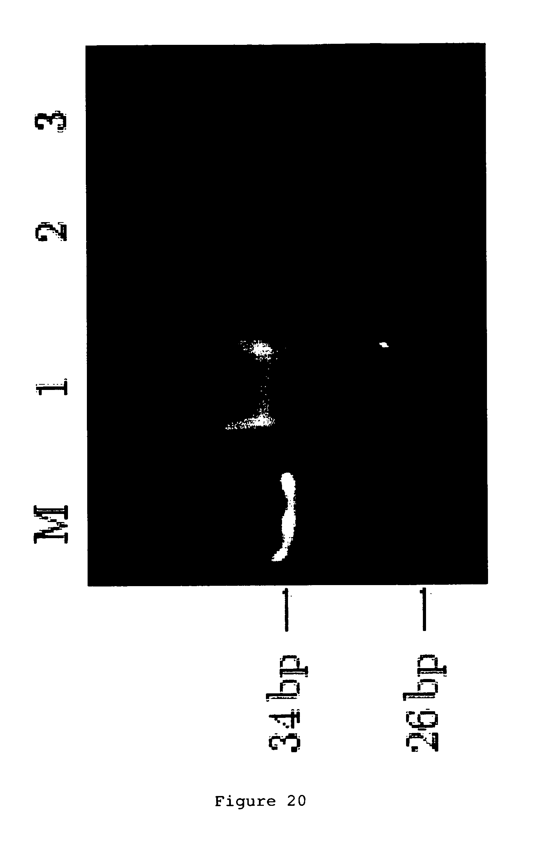

Enzymatical template-directed ligation of tetramer ssDNA into a gap with T4 RNA ligase: For ligation of tetramer ssDNA on a PNA template the oligonucleotides 4merLig5' (5'CAT TAG TTG GTG CAA3') and 4merLig3' (5'Pho-TAA TCG GGA TCT GAG3') (Figure 19; blank) where used which hybridized with seven nucleotides each on a PNA template (N-Bio-CCG ATT AAC GCT TGC ACC-C) (filled black) leaving a gap of four nucleotides. The gap was filled with suiting 5'-phosphorylated tetramer-ssDNA from a randomized library (4mer-deg; 5'Pho-NNN N3'). The reaction was performed in 50 mmol/l HEPES/NaOH (pH 8), 10 mmol/l magnesium chloride, 100 µmol/l ATP and 10 µg/ml BSA with 330 U/ml T4 RNA ligase. Concentrations of oligonucleotides were 10 nM for 4merLig5' and 4merLig3' and 1 µmol/l for 4mer-deg. All components of the reaction except T4 RNA ligase and BSA were heated to 94°C for 2 min and cooled slowly to 25°C. Ligation was started by addition of T4 RNA ligase and BSA and the mixtures were subsequently incubated at 25°C for 15 h. The reaction solution was transfered to a 200 µl streptavidin coated PCR tube and incubated 7 h at room temperature. After removal of the reaction solution the tube was washed twice with 200 µl washing buffer (0.2 mol/l NaCl, 10 mmol/l Tris-HCl (pH 7.5), 1 mmol/l EDTA, 0.1 % (v/v) Tween 20) and the DNA from immobilized PNA/DNA-hybrids was amplified by addition of a primer extension mix (20 mmol/l Tris-HCl, 10 mmol/l ammoniumsulfate, 10 mmol/l potassiumchloride, 2 mmol/l magnesium sulfate, 0.1 % Triton X-100, 200 µmol/l each dNTP and respectively 1 µmol/l of the primers 4merLig5' (5'CAT TAG TTG GTG CAA3') and 4merLig3'rev (5'CTC AGA TCC CGA TTA3')) and subsequent temperature cycling (96°C primary denaturation; 30 cycles of 96°C, 1 min; 38°C, 1 min; 72°C, 30 s; final elongation 72°C, 1 min). 34 base pair long amplification products were detected in a PAGE analysis of the amplification reaction with 15 % polyacrylamide under native conditions (Figure 20). The DNA was subcloned into appropriate vector DNA by T-overhang and sequenced subsequently. (lane 1: ligation with PNA; lane 2: ligation without PNA template; lane 3: negative control for PCR)

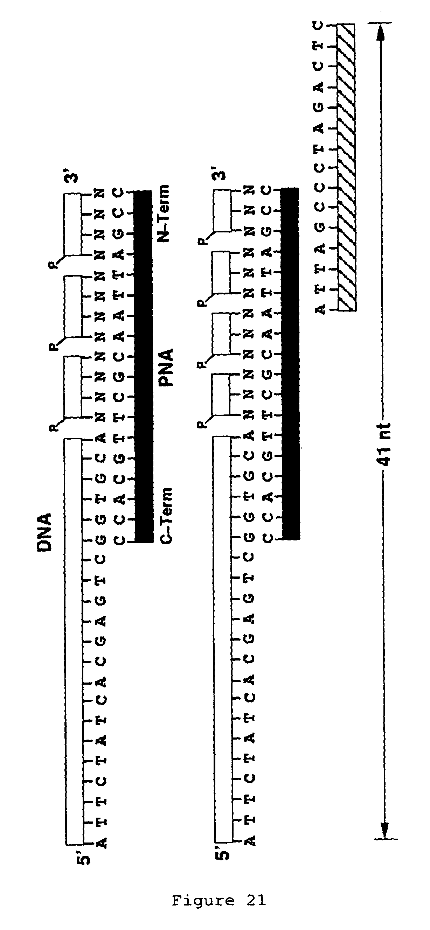

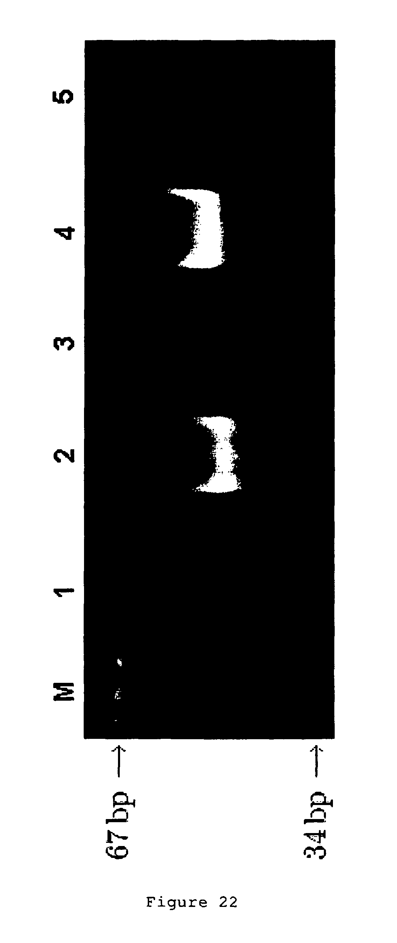

Template-directed elongation of PNA-hybridized ssDNA with tetramer- and trimer-ssDNA: Elongation of the oligonucleotide APos2(5)(-3)D2-lrev (5'ATT CTA TCA CGA GTC GGT GCA3') was performed using the PNA (N-CCG ATT AAC GCT TGC ACC-C) as template for ligation of 5'-phosphorylated 4mer- (4mer-deg; 5'Pho-NNN N3') or 3mer-ssDNA (3mer-deg; 5'Pho-NNN3') fragments (Figure 21). The reaction was performed in 50 mmol/l HEPES/NaOH (pH 8), 10 mmol/l manganese chloride, 100 µmol/l ATP, 20 mmol/l DTT, 2 mmol/l spermine, 10 µg/ml BSA with 330 U/ml T4 RNA ligase. Concentrations of the oligonucleotides were 0.8 µmol/l APos2(5)(-3)D2-lrev, 1 µmol/l PNA and 40 µmol/l of the oligonucleotide 4mer-deg respectively 69 µmol/l 3mer-deg. All components except T4 RNA Ligase and BSA were heated to 70°C for 2 min and cooled down to room temperature. Ligation reaction was started by addition of BSA and T4 RNA ligase. The sample were incubated at room temperature for 5 days. Ligation products were amplified in 50 µl PCR samples (20 mmol/l Tris-HCl, 10 mmol/l ammonium sulfate, 10 mmol/l potassium chloride, 2 mmol/l magnesium sulfate, 0.1 % Triton X-100, 200 µmol/l each dNTP and 1 µmol/l of each primer) containing 1 µl of the ligation reaction using the primers APos2(5) (-3)D2-1rev (5'ATT CTA TCA CGA GTC GGT GCA3') (blank) and 4merLig3'rev (hatched) (5'CTC AGA TCC CGA TTA3') (96°C primary denaturation; 10 cycles of 96°C, 2 min; 10°C, 5 min; 15°C, 5 min; 20°C, 5 min; 50°C, 1 min; 40 cycles of 96°C, 1 min; 40°C, 1 min; 72°C, 30 s; final elongation 72°C, 1 min) (P: phosphorylation; hatched: backward primer used for amplification of ligation products). The 41 base pair long amplification products were analysed in a PAGE with 15 % polyacrylamide under native conditions and 41 base pair long DNA could be detected (Figure 22). The DNA was subcloned into appropriate vector DNA by T-overhang and sequenced subsequently. It is obvious in Figure 22 that amplification products are exclusively obtained in samples which contained a PNA template (lanes 2 and 4). Althought only five nucleotides could be verified after sequencing, a strech of twelve nucleotides must have been generated in a template directed manner for a sucessfull PCR amplification with the used primers. (lane 1: negative control for PCR; lane 2: reaction with trimer ssDNA and PNA template; lane 3: reaction with trimer ssDNA without PNA template; lane 4: reaction with tetramer ssDNA and PNA template; lane 5: reaction with tetramer ssDNA without PNA template)

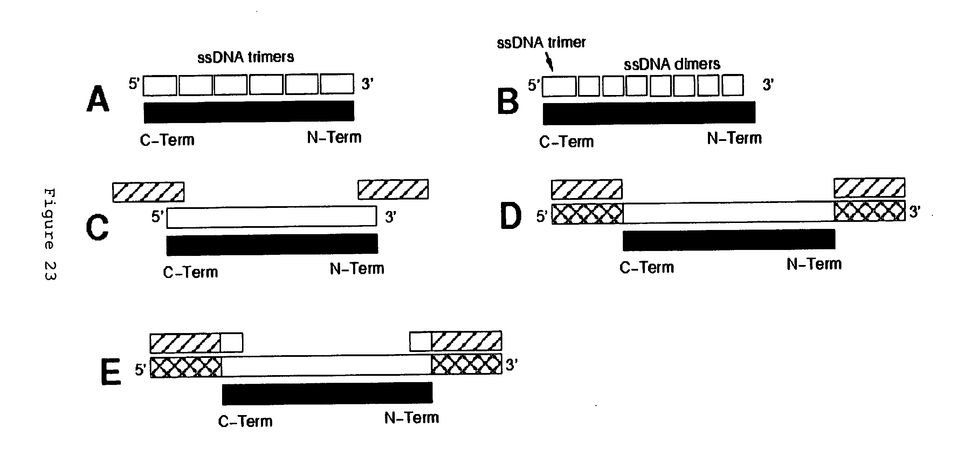

Template-directed synthesis of PNA-complementary DNA with ssDNA-fragments without overhang: Synthesis of PNA-complementary DNA can be performed by enzymatical or chemical coupling of ssDNA fragments with a length from nine to two nucleotides on a PNA template (Figure 23A). Whereas there is a need for a starter oligonucleotide with at least three nucleotides when using dimer fragments (Figure 23B).

Amplification of cDNA can be performed either by using primers which aim for the edges of the generated cDNA (Figure 23C) or by ligation of oligonucleotides that function as linkers to provide primer regions (Figure 23D). Amplification by directly joined linker oligonucleotides can be circumvented by using primers that are extended into the DNA (Fig 23E) that has been generated by known segments of the PNA.

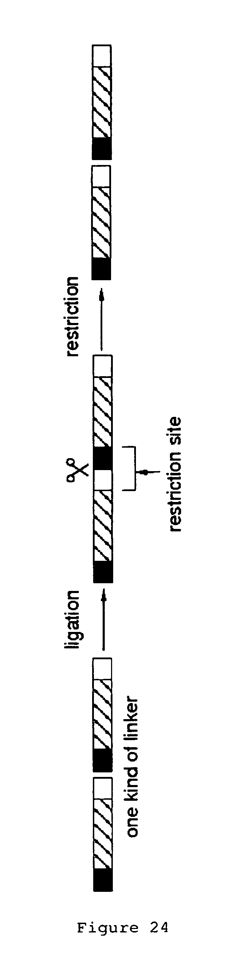

Restriction of directly ligated linker oligonucleotides by restriction endonucleases: Another method to prevent the amplification of directly joined linker oligonucleotides is their hydrolysis using nucleases (Figure 24). Only a single 5'-phophorylated oligonucleotide is used as linker and can be ligated at both ends of generated DNA. With the ligation of two linker oligonucleotides directly to each other, a restriction site for a restriction endonuclease is generated. After synthesis of a complementary strand by DNA polymerases, the fragments can be hydrolysed to prevent their amplification in a PCR.

Template-directed elongation of PNA-hybridized ssDNA with pyrophosphates: Synthesis of PNA-complementary DNA can be performed by elongation of an initiation nucleotide with a length of at least three nucleotides which hybridizes at the C-terminus of the PNA. Elongation can be executed by enzymatical and template-directed coupling of adenylated desoxyribonucleotides (A-5'pp5'-dN) or adenylated ribonucleotides (A-5'pp5'-N) that represent an intermediary product in the process of ligation ligation according to the reference England, T.E. et al., Dinucleoside pyrophosphates are substrates for T4-induced RNA ligase., Proc. Nat. Acad. Sci. 1977, 74(11), 4839-42.
(1) A-5'pp5'-dN + dNₙOH --> dNₙpdN + AMP (catalyzed by ligase) or (2) A-5'pp5'-N + NₙOH --> NₙpN + AMP (catalyzed by ligase)

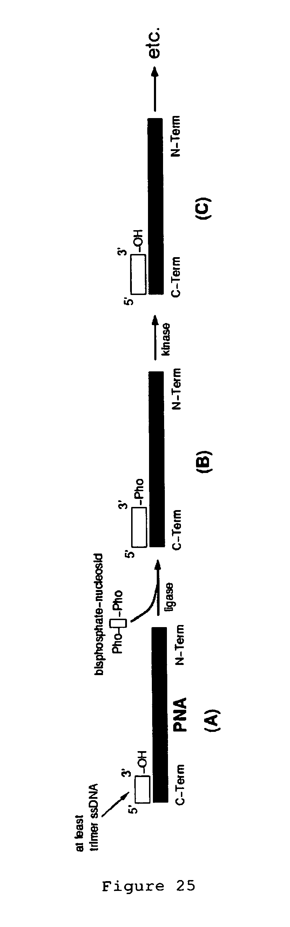

Template-directed elongation of PNA-hybridized ssDNA with 3',5'-bisphosphatenucleosides: Synthesis of PNA-complementary DNA can be performed by elongation of an initiation oligonucleotide with a length of at least three monomers which hybridizes at the C-terminus of the PNA. Elongation can be executed by template-directed enzymatical coupling of 2'-deoxynucleoside-3',5'-bisphosphates or nucleoside-3',5'-bisphosphates (Figure 25A). For proceeding elongation an intermediary dephosphorylation of resulting phosphorylated 3'-ends (B) is necessary. This can be realized for instance by kinases kinases (e.g. T4 polynucleotide kinase) (C).

Linear amplification of PNA-sequence information by ligase chain reaction: Usage of thermostable ligases offers the possibility of a linear amplification of complementary DNA on a PNA template. The synthesis of cDNA is executed by recurring changes of the temperature between a denaturating temperature to dissociate DNA from the PNA-template and an annealing and ligation temperature to hybridize and anneal ssDNA fragments on the template.

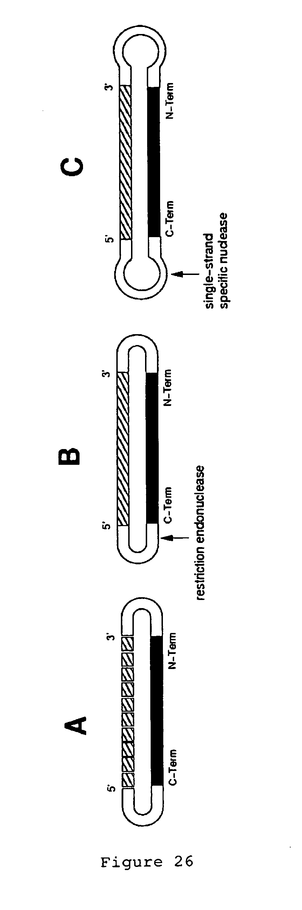

Using PNA/DNA-chimera with terminal DNA builing hairpin loops for ligation and to provide primer regions: Using PNA/DNA chimeras with flanking DNA segments (Figure 26A, blank) that form DNA hairpin loops at each terminus of the PNA (A, filled black) can serve as initiation oligonucleotides for the template-directed ligation of ssDNA fragments (A, hatched) on the PNA. Furthermore these nucleotides can provide primer regions for subsequent amplification of the ligation products after cutting either the stem regions with restriction enzymes (B) or single-stranded segments with single-strand specific nucleases (S1 nuclease, Mungobean nuclease etc.) (C).

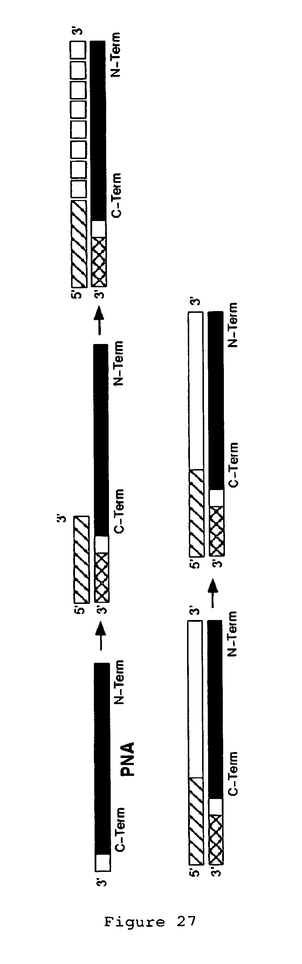

Using PNA/DNA-chimera with terminal DNA being elongated by terminal-nucleotidyl transferases to provide hybridization regions: Attached nucleotides (Figure 27; blank) at the C-terminus of a PNA (filled black) can be elongated with one kind of monomers using terminal nucleotidyl transferases. These generated DNA tails (cross-hatched) provide hybridization sites for initiation oligonucleotides (hatched) which can be elongated by ssDNA fragments (blank) using ligases. Additionally the generated DNA tails can serve as hybridization regions for primers (grey) for the subsequent amplification of ligation products by PCR.

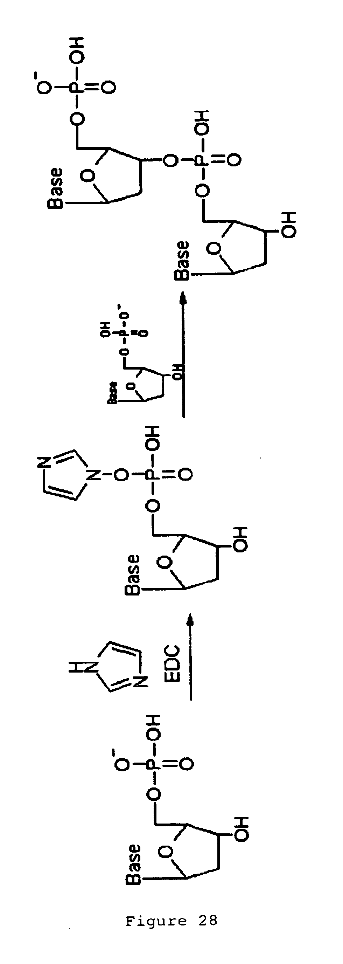

Using chemically preactivated buildings blocks or monomers: Ligation of ssDNA fragments or monomers on a PNA template can be performed by using chemically preactivated building-blocks. These building blocks can be generated for instance by activation of a phosphate group at the 5'- or 3'-end of ssDNA by using carbodiimides (e.g. EDC) and imidazole or NHS (Figure 28). Alternatively priming oligonucleotides hybridized at the egde of a PNA can be elongated by addition of chemically preactivated monomers or ssDNA fragments in a manner which has been mentioned above.

### Example 6: Obtaining PNA sequence information

After hybridization or generation of complementary ss nucleic acids according to example 5 the nucleic acids are sequenced according to the reference Sanger F. et al., DNA-sequencing with chain-terminating inhibitors., Proc. Nat. Acad. Sci. 1977, 74, 5463-5467 and Hunkapiller, T., Large-scale and automated DNA sequence determination., Science 1991, 254, 59-67. The sequence information obtained thereby is translated into complementary sequence information, which is the sequence information of the PNA. With this PNA sequence information PNA molecules with such sequence can be synthesized according to example 3.

## Claims

1. Method for determining an unknown PNA sequence information of PNA molecules of a specific PNA molecule species,
wherein, the PNA molecules are contacted with one or several different nucleic acid molecule species comprising nucleic acid molecules with at least one nucleotide, wherein the nucleic acid molecules at least partially comprise a nucleic acid sequence that is complementary to at least a partial sequence of the PNA molecule,
wherein nucleic acid molecules having complementary sequences bind to the PNA molecules forming nucleic acid/PNA hybrids,
wherein nucleic acid molecules with non-complementary sequences are separated from the nucleic acid/PNA hybrids and/or are degraded enzymatically,
wherein thereafter the nucleic acid/PNA hybrids are dissociated into single stranded hybrid nucleic acid molecules and PNA molecules,
wherein the single stranded hybrid nucleic acid molecules are subjected to a sequencing process providing hybrid sequence information about the single stranded hybrid nucleic acid sequence, and
wherein the hybrid sequence information is optionally translated into the complementary PNA sequence information.

2. Method according to claim 1, wherein the PNA is contacted with a plurality of nucleic acid species comprised in a randomized nucleic acid library.

3. Method according to claim 1 or 2, wherein the sequence length of the PNA is at least 5, preferably at least 10, more preferably at least 15.

4. Method according to claim 1 or 2, wherein the sequence length of the nucleic acid molecules is at least 2, preferably at least 3, more preferably at least 4.

5. Method according to any of the claims 1 to 4, wherein the sequence length of the nucleic acid molecules is at least the sequence length of the PNA molecules, and wherein binding of complementary PNA molecules and nucleic acid molecules is carried out under hybridization conditions forming the nucleic acid/PNA hybrids without ligation of nucleic acid molecules bound to the PNA molecules.

6. Method according to any of the claims 1 to 4, wherein the sequence length of the PNA molecules is greater than a total sequence length or a randomized partial sequence length of the nucleic acid molecules, preferably by a factor of more than 2, more preferably by a factor of more than 3, most preferably by a factor of more than 4, and wherein nucleic acid molecules binding adjacent to each other to the PNA molecules are ligated chemically or enzymatically forming the nucleic acid/PNA hybrid.

7. Method according to claim 6, wherein the ligation is carried out enzymatically using a ligation enzyme selected from the group consisting of "DNA ligase I, DNA ligase II, DNA ligase III, DNA ligase IV, DNA ligase V, T4 DNA ligase, Taq DNA ligase, T4 RNA ligase, T4 RNA ligase II, ThermoPhage^{™} single-stranded DNA ligase, Rma DNA ligase, Tsc DNA ligase, *E.coli* DNA ligase, LdMNPV DNA ligase, LigTK, Mth ligase, PBCV-1 DNA ligase, Pfu DNA ligase, Sealase, T4 ATP ligase, Vaccinia ligase, Tfi DNA ligase, Tth DNA ligase, Band IV, DREL, gp24.1, P52, RM378 RNA ligase, TbMP52, Rcl1p, DNA ligase D, XRCC4-ligase, T7 DNA ligase, Bst ligase, DraRnl", preferably using T4 RNA ligase.

8. Method according to any of the claims 1 to 7, wherein the single stranded hybrid nucleic acid molecules are amplified, preferably using PCR, prior to the sequencing process.

9. Method for selecting and/or identifying PNA molecules of a PNA molecule species forming PNA/target molecule complexes with target molecules of a target molecule species comprising the following steps:
a) a solution comprising a PNA molecule library with PNA molecules of a plurality of different PNA molecule species is contacted with target molecules of at least one target molecule species,
b) the solution obtained in step a) and comprising unbound PNA molecules and PNA/target molecule complexes is optionally subjected to a separation method, wherein unbound PNA molecules are separated from the PNA/target molecule complexes and unbound PNA molecules are discarded,
c) the PNA/target molecule complexes obtained in step b) are dissociated to PNA molecules and target molecules, and the PNA molecules are optionally separated from the target molecules,
d) the PNA molecules obtained in step c) are contacted with nucleic acid molecules of a plurality of different nucleic acid species, wherein the PNA molecules hybridize with nucleic acid molecules having a nucleic acid sequence being complementary to the sequence of the PNA, wherein the non-hybridized nucleic acid molecules are optionally separated from hybridized nucleic acid molecules by physical and/or chemical methods and/or preferably are degraded by enzymatical methods,
e) the hybridizing nucleic acid molecules obtained in step d) are optionally amplified,
f) the optionally amplified hybridizing nucleic acid molecules obtained in step e) are subjected to a sequencing process, optionally after cloning, wherein nucleic acid sequence information about the sequence of the amplified nucleic acid molecules obtained in step e) is generated,
g) for identifying, the nucleic acid sequence information obtained in step f) is translated to complementary PNA sequence information,

## Patentansprüche

1. Verfahren zur Bestimmung von unbekannten PNA-Sequenzinformationen von PNA-Molekülen einer bestimmten PNA-Molekülspezies,
wobei die PNÄ-Moleküle mit einer oder mehreren verschiedenen Nukleinsäure-Molekülspezies mit Nukleinsäure-Molekülen mit wenigstens einem Nukleotid kontaktiert werden, wobei die Nukleinsäure-Moleküle wenigstens teilweise eine Nukleinsäure-Sequenz aufweisen, die zu wenigstens einer Teilsequenz des PNA-Moleküls komplementär ist,
wobei die Nukleinsäure-Moleküle mit komplementären Sequenzen sich an die PNA-Moleküle unter Bildung von Nukleinsäure/PNA-Hybriden binden,
wobei die Nukleinsäure-Moleküle mit nicht-komplementären Sequenzen von den Nukleinsäure/PNA-Hybriden getrennt und/oder enzymatisch zersetzt werden,
wobei danach die Nukleinsäure/PNA-Hybride in einsträngige Hybrid-Nukleinsäure-Moleküle und PNA-Moleküle zersetzt werden,
wobei die einsträngigen Hybrid-Nukleinsäure-Moleküle einem Sequenzierungsprozess unterworfen werden, was Hybrid-Sequenzinformationen über die einsträngige Hybrid-Nukleinsäure-Sequenz liefert, und
wobei die Hybrid-Sequenzinformationen optional in die komplementären PNA-Sequenz-Informationen umgesetzt werden.

2. Verfahren nach Anspruch 1, wobei die PNA mit einer Vielzahl von in einer randomisierten Nukleinsäurebibliothek enthaltenen Nukleinsäurespezies kontaktiert wird.

3. Verfahren nach Anspruch 1 oder 2, wobei die Sequenzlänge der PNA wenigstens 5, vorzugsweise wenigstens 10, höchst vorzugsweise wenigstens 15 ist.

4. Verfahren nach Anspruch 1 oder 2, wobei die Sequenzlänge der Nukleinsäure-Moleküle wenigstens 2, vorzugsweise wenigstens 3, höchst vorzugsweise wenigstens 4 ist.

5. Verfahren nach einem der Ansprüche 1 bis 4, wobei die Sequenzlänge der Nukleinsäure-Moleküle wenigstens gleich der Sequenzlänge der PNA-Moleküle ist, und wobei die Bindung der komplementären PNA-Moleküle und Nukleinsäure-Moleküle unter Hybridisierungsbedingungen ausgeführt wird, wodurch die Nukleinsäure/PNA-Hybride ohne Ligation der an die PNA-Moleküle gebundenen Nukleinsäure-Moleküle gebildet werden.

6. Verfahren nach einem der Ansprüche 1 bis 4, wobei die Sequenzlänge der PNA-Moleküle größer als eine Gesamtsequenzlänge oder eine randomisierte Teilsequenzlänge der Nukleinsäure-Moleküle ist, vorzugsweise um einen Faktor von mehr als 2, höher vorzugsweise um einen Faktor von mehr als 3, höchst vorzugsweise um einen Faktor von mehr als 4, und wobei sich benachbart zueinander an die PNA-Moleküle bindende Nukleinsäure-Moleküle chemisch oder enzymatisch ligiert werden, wodurch der Nukleinsäure/PNA-Hybrid gebildet wird.

7. Verfahren nach Anspruch 6, wobei die Ligation enzymatisch ausgeführt wird unter Verwendung eines Ligationsenzyms ausgewählt aus der Gruppe bestehend aus "DNA-Ligase I, DNA-Ligase II, DNA-Ligase III, DNA-Ligase IV, DNA-Ligase V, T4-DNA-Ligase, Taq-DNA-Ligase, T4-RNA-Ligase, T4-RNA-Ligase II, ThermoPhage einsträngige DNA-Ligase, Rma-DNA-Ligase, Tsc-DNA-Ligase, E.coli-DNA-Ligase, LdMNPV-DNA-Ligase, LigTK, Mth-Ligase, PBCV-1-DNA-Ligase, Pfu-DNA-Ligase, Sealase, T4-ATP-Ligase, Vaccinia-Ligase, Tfi-DNA-Ligase, Tth-DNA-Ligase, Band IV, DREL, gp24.1, P52, RM378 RNA-Ligase, TbMP52, Rc11p, DNA-Ligase D, XRCC4-Ligase, T7-DNA-Ligase, Bst-Ligase, DraRn1", vorzugsweise unter Verwendung von T4-RNA-Ligase.

8. Verfahren nach einem der Ansprüche 1 bis 7, wobei die einsträngigen Hybrid-Nukleinsäure-Moleküle amplifiziert werden, vorzugsweise unter Verwendung von PCR, vor dem Sequenzierungsprozess.

9. Verfahren zum Auswählen und/oder Identifizieren von PNA-Molekülen einer PNA/Zielmolekül-Komplexe bildenden PNA-Molekülspezies mit Zielmolekülen einer Zielmolekülspezies mit den folgenden Schritten:
a) eine Lösung mit einer PNA-Molekül-Bibliothek mit PNA-Molekülen einer Vielzahl von verschiedenen PNA-Molekülspezies wird mit Zielmolekülen wenigstens einer Zielmolekülspezies kontaktiert,
b) die in Schritt a) erhaltene und ungebundene PNA-Moleküle und PNA/Zielmolekül-Komplexe enthaltende Lösung wird optional einem Trennverfahren unterworfen, wobei ungebundene PNA-Moleküle von den PNA/Zielmolekül-Komplexen getrennt werden, und ungebundene PNA-Moleküle verworfen werden,
c) die in Schritt b) erhaltenen PNA/Zielmolekül-Komplexe werden in PNA-Moleküle und Zielmoleküle getrennt, und die PNA-Moleküle werden optional von den Zielmoleküle separiert,
d) die in Schritt c) erhaltenen PNA-Moleküle werden mit Nukleinsäure-Molekülen einer Vielzahl von verschiedenen Nukleinsäurespezies kontaktiert, wobei die PNA-Moleküle mit Nukleinsäure-Molekülen hybridisieren, die eine zur Sequenz der PNA komplementäre Nukleinsäure-Sequenz aufweisen, wobei die nicht-hybridisierten Nukleinsäure-Moleküle optional von den hybridisierten Nukleinsäure-Molekülen durch physikalische und/oder chemische Verfahren separiert werden und/oder vorzugsweise durch enzymatische Verfahren zersetzt werden,
e) die in Schritt d) erhaltenen hybridisierenden Nukleinsäure-Moleküle werden optional amplifiziert,
f) die in Schritt e) erhaltenen optional amplifizierten hybridisierenden Nukleinsäure-Moleküle werden einem Sequenzierungsprozess unterworfen, optional nach Klonen, wobei Nukleinsäure-Sequenzinformationen über die Sequenz der in Schritt e) erhaltenen amplifizierten Nukleinsäure-Moleküle erzeugt werden,
g) zum Identifizieren werden die in Schritt f) erhaltenen Nukleinsäure-Sequenzinformationen in komplementäre PNA-Sequenzinformationen umgesetzt.

## Revendications

1. Procédé pour déterminer des informations de séquence ANP inconnues de molécules d'ANP d'une espèce de molécule d'ANP spécifique,
dans lequel les molécules d'ANP sont contactées avec une ou plusieurs espèces de molécule d'acide nucléique différentes comprenant des molécules d'acide nucléique avec au moins un nucléotide, dans lequel les molécules d'acide nucléique au moins en partie comprennent une séquence d'acide nucléique, qui est complémentaire à au moins une séquence partielle de la molécule d'ANP,
dans lequel les molécules d'acide nucléique ayant des séquences complémentaires se lient aux molécules d'ANP formant des hybrides d'acide nucléique/ANP,
dans lequel les molécules d'acide nucléique avec des séquences non-complémentaires sont séparées des hybrides d'acide nucléique/ANP et/ou sont dégradées enzymatiquement,
dans lequel puis les hybrides d'acide nucléique/ANP sont dissociés en des molécules hybrides d'acide nucléique à un brin et des molécules d'ANP,
dans lequel les molécules hybrides d'acide nucléique à un brin sont soumises à un procédé de séquentiation fournissant des informations de séquence hybride sur la séquence hybride d'acide nucléique à un brin, et
dans lequel les informations de séquence hybride sont optionnellement traduites en des informations de séquence d'ANP complémentaire.

2. Procédé selon la revendication 1, dans lequel l'ANP est contacté avec une pluralité d'espèces d'acide nucléique comprise dans une bibliothèque d'acides nucléiques randomisés.

3. Procédé selon la revendication 1 ou 2, dans lequel la longueur de la séquence de l'ANP est au moins 5, de préférence au moins 10, de préférence plus haute au moins 15.

4. Procédé selon la revendication 1 ou 2, dans lequel la longueur de la séquence des molécules d'acide nucléique est au moins 2, de préférence au moins 3, de préférence plus haute au moins 4.

5. Procédé selon une des revendications 1 à 4, dans lequel la longueur de la séquence des molécules d'acide nucléique est au moins comme la longueur de la séquence des molécules d'ANP, et dans lequel le liage de molécules d'ANP complémentaires et de molécules d'acide nucléique est exécuté sous des conditions d'hybridation formant les hybrides d'acide nucléique/ANP sans ligation de molécules d'acide nucléique liées aux molécules d'ANP.

6. Procédé selon une des revendications 1 à 4, dans lequel la longueur de la séquence des molécules d'ANP est supérieure à une longueur de la séquence totale ou une longueur de séquence partielle randomisée des molécules d'acide nucléique, de préférence par un facteur de plus de 2, préférence plus haute par un facteur de, plus de 3, de préférence la plus haute par un facteur de plus de 4, et dans lequel les molécules d'acide nucléique se liant voisine l'une à l'autre aux molécules d'ANP sont liguées chimiquement ou enzymatiquement en formant l'hybride d'acide nucléique/ANP.

7. Procédé selon la revendication 6, dans lequel la ligation est exécutée enzymatiquement en utilisant une enzyme de ligation choisie à partir du groupe comprenant "ADN ligase I, ADN ligase II, ADN ligase III, ADN ligase IV, ADN ligase V, T4 ADN ligase, Taq ADN ligase, T4 RNA ligase, T4 RNA ligase II, ThermoPhage ADN ligase à un brin, Rma ADN ligase, Tsc ADN ligase, E.coli ADN ligase, LdMNPV ADN ligase, LigTK, Mth ligase, PBCV-1 ADN ligase, Pfu ADN ligase, Sealase, T4 ATP ligase, Vaccinia ligase, Tfi ADN ligase, Tth ADN ligase, Band IV, DREL, gp24.1, P52, RM378 RNA ligase, TbMP52, Rc11p, ADN ligase D, XRCC4-ligase, T7 ADN ligase, Bst ligase, DraRn1", de préférence en utilisant la T4 RNA ligase.

8. Procédé selon une des revendications 1 à 7, dans lequel les molécules hybrides d'acide nucléique à un brin sont amplifiées, de préférence en utilisant PCR, avant le procédé de séquentiation.

9. Procédé pour choisir et/ou identifier des molécules d'ANP d'une espèce de molécules d'ANP formant des complexes de molécule d'ANP/cible avec des molécules cibles d'une espèce de molécules cibles comprenant les étapes suivantes:
a) une solution comprenant une bibliothèque de molécules d'ANP avec des molécules d'ANP d'une pluralité d'espèces de molécules d'ANP différentes est contactée avec des molécules cibles d'au moins une espèce de molécule cible,
b) la solution obtenue dans l'étape a) et comprenant des molécules d'ANP non-liées et des complexes de molécules ANP/cible est optionnellement soumise à un procédé de séparation, dans lequel des molécules d'ANP non-liées sont séparées des complexes de molécules ANP/cible et des molécules d'ANP non-liées sont rejetées,
c) les complexes de molécules d'ANP/cible obtenus dans l'étape b) sont dissociés en des molécules d'ANP et molécules cibles, et les molécules d'ANP sont optionnellement séparées des molécules cibles,
d) les molécules d'ANP obtenues dans l'étape c) sont contactées avec les molécules d'acide nucléique d'une pluralité d'espèces d'acide nucléique différentes, dans lequel les molécules d'ANP'hybrid,ent avec les molécules d'acide nucléique ayant une séquence d'acide nucléique étant complémentaire à la séquence de l'ANP, dans lequel les molécules d'acide nucléique non-hybridées sont optionnellement séparées des molécules d'acide nucléique hybridées par des procédés physiques et/ou chimiques et/ou de préférence sont dégradées par des procédés enzymatiques,
e) les molécules d'acide nucléique hybridantes obtenues dans l'étape d) sont optionnellement amplifiées,
f) les molécules d'acide nucléique hybridantes optionnellement amplifiées obtenues dans l'étape e) sont soumises à un procédé de séquentiation, optionnellement après un clonage, dans lequel les informations de séquence d'acide nucléique sur la séquence des molécules d'acide nucléique amplifiées obtenues dans l'étape e) sont générées,
g) pour identifier, les informations de séquence d'acide nucléique obtenues dans l'étape f) sont traduites en des informations de séquence d'ANP complémentaires.
